Europäisches Patentamt
European Patent Office
Office européen des brevets

(19)

(11) Publication number: **0 173 377 A2**

(12) # EUROPEAN PATENT APPLICATION

(21) Application number: **85201233.5**

(22) Date of filing: **11.02.83**

(51) Int. Cl.[4]: **C 07 D 239/36**, C 07 D 405/12, C 07 D 417/12, C 07 C 123/00, C 07 C 69/716, C 07 C 69/738, A 61 K 31/505, C 07 D 417/14, C 07 D 405/14, C 07 D 401/06, C 07 D 403/06

(30) Priority: **15.02.82 JP 22280/82**
**26.08.82 JP 148123/82**
**27.08.82 JP 148967/82**
**01.09.82 JP 151997/82**
**13.12.82 JP 218209/82**

(43) Date of publication of application: **05.03.86 Bulletin 86/10**

(84) Designated Contracting States: **AT BE CH DE FR GB IT LI LU NL SE**

(60) Publication number of the earlier application in accordance with Art. 76 EPC: **0086647**

(71) Applicant: **YAMANOUCHI PHARMACEUTICAL CO. LTD., No. 5-1 Nihonbashi-Honcho, 2-chome Chuo-ku, Tokyo (JP)**

(72) Inventor: **Yanagisawa, Isao, 2-22-8, Shakujiidai Nerima-ku, Tokyo (JP)**
Inventor: **Ohta, Mitsuaki, 3-16-1, Hasune Itabashi-ku, Tokyo (JP)**
Inventor: **Takagi, Tokuichi, No. 8-27-13, Takasago Katsushika-ku, Tokyo (JP)**

(74) Representative: **Geering, Keith Edwin et al, REDDIE & GROSE 16 Theobalds Road, London WC1X 8PL (GB)**

(54) **Pyrimidone compounds, their preparation and pharmaceutical compositions containing them.**

(57) Novel pyrimidones of the formula I and salts thereof are gastric acid secretion inhibitors and some have other medical utility:

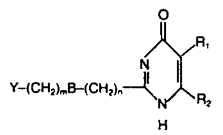

(I)

wherein $R_1$ represents a hydrogen atom, a $C_1$ to $C_{10}$ alkyl group, a $C_1$ to $C_5$ alkenyl group, a $C_1$ to $C_5$ alkinyl group, a phenyl group, a cycloalkylmethyl group, or an unsubstituted or substituted aralkyl group; $R_2$ represents a hydrogen atom, a $C_1$ to $C_5$ alkyl group or a trifluoromethyl group; Y represents a heterocyclic group of the formula:

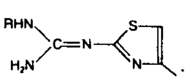 , 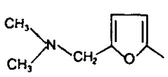 or 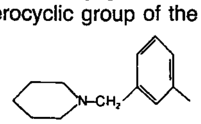

wherein R represents a hydrogen atom or a $C_1$ to $C_5$ alkyl group; B represents an oxygen atom or a sulfur atom: m represents zero or an integer of 1 to 3; and n represents an integer of 1 to 3. Methods and novel intermediates for their preparation are disclosed, as well as medicaments containing them and medical treatments using them.

EP 0 173 377 A2

# PYRIMIDONE COMPOUNDS, THEIR PREPARATION AND PHARMACEUTICAL COMPOSITIONS CONTAINING THEM

The present invention relates to pyrimidone compounds useful in general as gastric acid secretion inhibitors, their preparation and intermediates therefor, and medical compositions containing them. Some compounds of this invention are also useful as anti-inflammatory agents or medicaments for treating disease of the cardio-vascular system or disease of the stomach.

Known pyrimidone compounds include those of the formula :

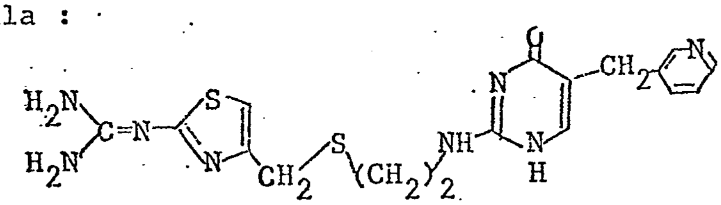

which exhibit prolonged gastric acid secretion inhibiting activity (published Japanese Patent Application No: 55-115883); and those of the formula :

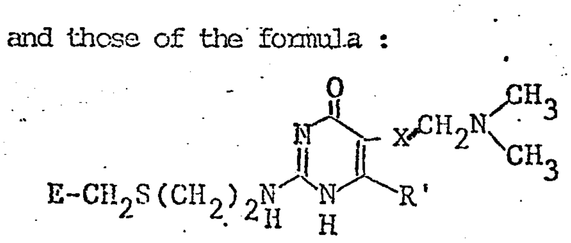

where E is

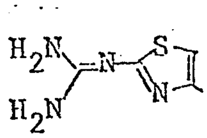

or

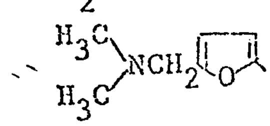

and X is $-CH_2$-heterocyclic- ,

which have the characteristic of a dimethylaminomethyl group at the 5-position. Other pyrimidone derivatives

are disclosed in published Japanese patent applications Nos. 54-115385 and 55-500898.

According to this invention there are provided pyrimidone compounds of general formula I :

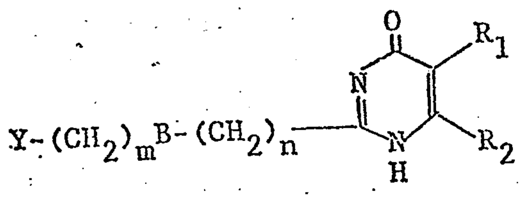

I

wherein $R_1$ represents a hydrogen atom, an alkyl group, a lower alkenyl group, a lower alkinyl group, a phenyl group, a cycloalkylmethyl group, an unsubstituted or substituted aralkyl group, or the group 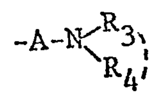 wherein A represents a lower alkylene group and $R_3$ and $R_4$ are the same or different lower alkyl groups or combine to form with the adjacent nitrogen atom a piperidine or pyrrolidine ring; $R_2$ represents a hydrogen atom, a lower alkyl group or a trifluoromethyl group; Y represents a heterocyclic group of the formula :

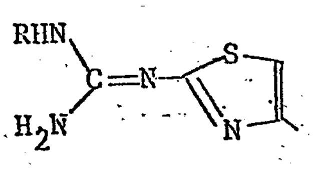 , 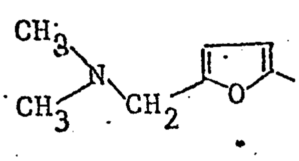 or 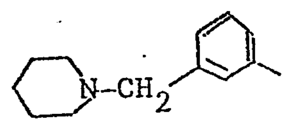

wherein R represents a hydrogen atom or a lower alkyl group; B represents an oxygen atom or a sulfur atom; m represents zero or an integer of 1 to 3; and n represents an integer of 1 to 3; and the pharmacologically acceptable salts thereof.

The invention also provides processes and intermediates or starting compounds for preparing compounds of general formula I, methods of treatment by administering effective amounts of formula I compounds, and pharmaceutical compositions containing effective amounts of formula I compounds and pharmaceutically acceptable carrier(s) and/or diluent(s).

The term "lower" in the above definition means a straight or branched carbon chain having 1 to 5 carbon atoms. For example, as lower alkyl groups, there are methyl, ethyl, propyl, isopropyl, butyl, isobutyl, sec-bytyl, tert-butyl, and pentyl groups etc; as lower alkenyl groups there are vinyl and allyl groups etc; as lower alkinyl groups there are ethinyl, 2-propinyl, 3-butinyl, 2-butinyl, 1-butinyl, 1-methyl-2-propinyl, and pentinyl groups etc.; and as lower alkylene groups there are methylene, ethylene, methylmethylene, trimethylene, propylene, tetramethylene, methyltrimethylene, and pentamethylene groups etc.

The alkyl group in the definition of $R_1$ is a straight or branched alkyl group having 1 to 10 carbon atoms. For example, as the alkyl group, there are, in addition to the above listed lower alkyl groups, n-hexyl, isohexyl, 1-methylpentyl, 2-methylpentyl, 3-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,2,2-trimethylpropyl, 1-ethyl-2-methylpropyl, 1-ethyl-1-methylpropyl,

n-heptyl, 1-methylhexyl, 2-methylhexyl, 3-methylhexyl, 4-methylhexyl, 5-methylhexyl, 1,1-dimethylpentyl, 1,2-dimethylpentyl, 1,3-dimethylpentyl, 1,4,dimethylpentyl, 2,2-dimethylpentyl, 2,3-dimethylpnentyl, 2,4-dimethylpentyl, 1-ethlylpentyl, 2-ethylpentyl, 3-ethylpentyl, 1-ethyl-1-methylbutyl, 1-ethyl-2-methylbutyl, 1-ethyl-3-methylbutyl, 2-ethyl-1-methylbutyl, 2-ethyl-2-methylbutyl, 1,1,2-trimethylbutyl, 1,2,2-trimethylbutyl, 1,2,3-trimethylbutyl, 1-propylbutyl, n-octyl, 6-methylheptyl, n-nonyl, 7-methyloctyl, n-desyl, and 8-methylnonyl groups, etc.

When $R_1$ is an aralky group, the aryl portion may for example be a phenyl or naphthyl group, a 5 or 6 membered heterocyclic group containing nitrogen atom(s) such as a pyridyl, pyrazolyl or imidazolyl group etc., or other unsaturated heterocyclic group containing nitrogen atom(s) such as a benzimidazolyl, indazolyl or quinolyl group etc, Such aryl groups may have one or more substituents such as lower alkyl, halogen, lower alkoxy, lower alkylenediosy, phenyl etc. As such halogens there are fluorine, chlorine, bromine etc.; as such alkoxy groups, there are for example methoxy, ethoxy, propoxy and butoxy groups etc.; and as such lower alkylenedioxy groups there are for example methylenedioxy and ethylenedioxy groups etc.

As the above aryl group, there are particularly listed naphthyl, pyridyl, benzimidazolyl, and phenyl optionally substituted by one or more substituent(s) selected from halogens and tert-butyl, methylenedioxy and phenyl groups.

As cycloalky groups herein there are for example cyclohexyl and cyclopentyl groups etc.

Typical compounds of this invention are as follows:

5-(p-tert-butylbenzyl)-2-[2-[[[2-[(diaminomethylene)amino]-4-thiazolyl]methyl]thio]ethyl]-6-methyl-4(1H)-pyrimidone;

2-[2-[[[2-[(diaminomethylene)amino]-4-thiazolyl]methyl]thio]-ethyl]-5-n-butyl-6-methyl-4(1H)-pyrimidone;

2-[2-[[[2-[(diaminomethylene)amino]-4-thiazolyl]methyl]thio]-ethyl]-6-methyl-5-n-pentyl-4(1H)-pyrimidone;

2-[2-[[[2-[(diaminomethylene)amino]-4-thiazolyl]methyl]thio]-ethyl]-6-methyl-5-(1-naphthylmethyl)-4(1H)-pyrimidone;

2-[2-[[[2-[(diaminomethylene)amino]-4-thiazolyl]methyl]thio]-ethyl]-5-(3-dimethylaminopropyl)-6-methyl-4(1H)-pyrimidone;

2-[2-[[[2-[(diaminomethylene)amino]-4-thiazolyl]methyl]-thio]ethyl]-5-(3-diethylaminopropyl)-6-methyl-4(1H)-pyrimidone;

5-[(1,3-benzodioxole-5-yl)methyl]-2-[2-[5-(dimethylaminomethyl)furfurylthio]ethyl]-6-methyl-4(1H)-pyrimidone; and

5-(p-tert-butylbenzyl)-6-methyl-2-[3-(m-piperidinomethyl-phenoxy)propyl]-4(1H)-pyrimidone.

The compounds of formula I can form acid addition salts, with mineral acids such as hydrochloric acid, hydrobromic acid, hydroiodic acid and sulfuric acid, etc., and with organic acids such as maleic acid, fumaric acid, and picric acid, etc. Furthermore, there also exist tautomers of the compounds of formula I at the 1H- and 3H-positions, and there exist occasionally hydroxy-tautomers in a small proportion. The invention includes such salts and tautomers.

The compounds of formula I and their acid addition salts have histamine $H_2$-receptor blocking activity, and are useful as gastric acid secretion inhibitors; some additionally have histamine $H_1$-receptor blocking activity and are useful also as anti-inflammatory agents and as medicaments acting on the cardio-vascular system.

Further, some compounds of this invention have cytoprotective action, and such compounds are useful as medicaments, for treating various diseases of the stomach.

For many of the compounds of this invention, their activities, such as histamine $H_2$-receptor blocking activity and/or histamine $H_1$-receptor blocking activity, are prolonged, lasting for a long period of time.

The compounds of this invention can be administered orally or parenterally, as the free bases or pharmacologically acceptable salts thereof. They are generally used as medical or pharmaceutical compositions with suitable carriers or diluents. The rate of administration is usually 100 to 800 mg per day for adults in 1 to 4 doses.

It has been shown by Shay rat 4 hr method (Shay et al., Gastroenterology, 5, pages 43 - 61, 1945) and other animal experiment methods that compounds of this invention inhibit gastric acid secretion at administered doses below 10 - 50 mg/kg and have histamine $H_2$-receptor blocking activity; that some of the compounds also have histamine $H_1$-receptor blocking activity; and that some also have cytoprotective action. Some relevant data is shown in the following Table :

Table

| Compound | $H_2$-blocking activity $ED_{50}$ | Acid Inhibition Shay (4hr.) (mg./kg.) (Dog) |
|---|---|---|
| 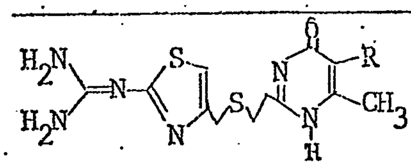 | | |
| R=methyl | $3.7x10^{-7}$ | |
| R=ethyl | $1.4x10^{-7}$ | 90% (0.1mg) |
| R=propyl | $1.7x10^{-7}$ | 60% (0.1mg) |
| R=CH≡CCH$_2$- | $1.8x10^{-7}$ | 95% (0.1mg) |
| R=butyl* | $1.5x10^{-7}$ | 70% (0.1mg) |
| R=amyl* | $0.9x10^{-7}$ | 95% (0.1mg) |
| R=iso-amyl* | $3x10^{-7}$ | + |
| R= p-tert-butyl-benzyl | $2.7x10^{-7}$ | |
| R= dimethylamino-propyl | $3.4x10^{-7}$ | |
| R= diethylamino-propyl | $5x10^{-7}$ | 83% (3mg) |
| 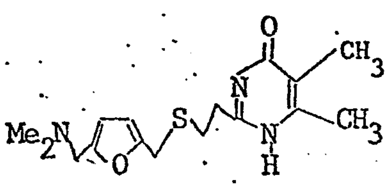 | $7.7x10^{-7}$ | |
| 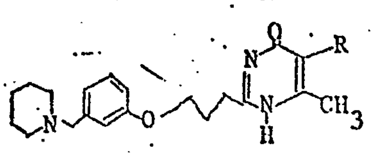 | | |
| R=tert-butylbenzyl | $5x10^{-7}$ | |
| R=H | $4.9x10^{-7}$ | |

* These compounds also have cytoprotective action.

Most at least of the compounds of this invention wherein $R_1$ is an alkyl group having 4 to 9 carbon atoms possess not only histamine $H_2$-receptor blocking activity but also cytoprotective action, and may therefor be useful as medicaments for treating various stomach diseases.

Compounds of this invention can be produced by various processes, some of which are shown below :

Process I.

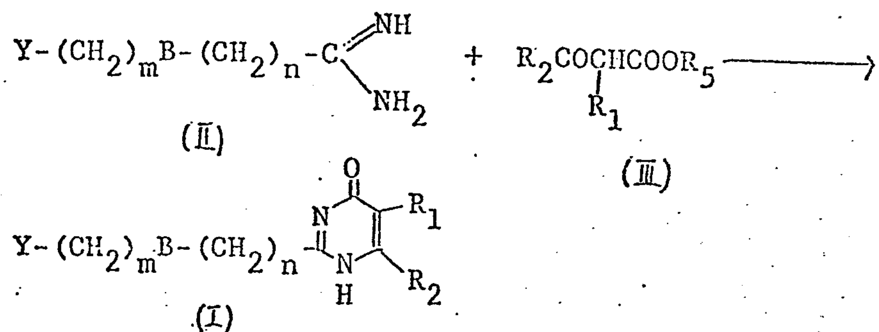

(II) (III)

(I)

(wherein $R_5$ represents a lower alkyl group)

This process is a condensation cyclization reaction between the amidine compound of formula (II) and the acylacetic acid derivative of formula (III).

The reaction is usually performed in a solvent at room temperature or under heating. As the solvent, there is preferably used alcohol (e.g. methanol, ethanol, isopropanol, etc), dimethylformamide, dimethylsulfoxide, methylcellosolve, ethylcellosolve, diglyme, etc. If necessary, a basic material may be present, suitable base materials including alkali metal (e.g. sodium or potassium) alcoholate, sodium hydroxide etc.

The starting materials (II) and (III) may be used in equival molar amounts, or some excess of either may be used.

Process II

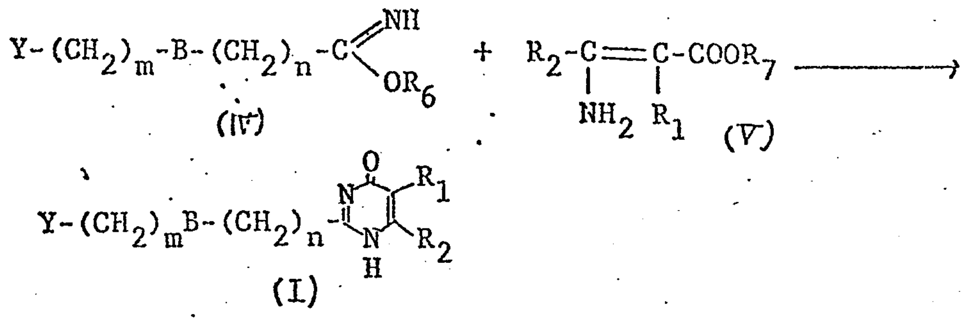

(IV) (V)

(I)

(wherein $R_6$ and $R_7$ are the same or different alkyl groups).

This process is a condensation cyclization reaction between the imidate compound of formula IV and the 3-aminoacrylic acid ester derivative of formula V. The reaction can be performed substantially as for Process I - i.e. using the same organic solvents and if necessary the same basic materials and reacting with heating or under reflux.

The compounds of this invention thus prepared can be separated as the free base or an acid addition salt thereof, and further can be purified by standard procedures such as column chromatography, recrystallization, etc.

The starting materials of formula II and IV can be obtained as disclosed in published Japanese patent applications Nos.55-115860, 55-115877 and 55-118476. Some of the starting materials of formula II

and III are novel, and can be produced as described in the Reference Examples 1 to 44 below.

Compounds of formula I and salts thereof, amd their preparation, are illustrated by Examples 1 to 64.

In the Examples and Reference Examples, mp., Anal., NMR and Mass. are abbreviations for melting point, elementary analysis values, nuclear magnetic resonance spectrum and mass spectrum, respectively.

Reference Example 1

Ethyl α-(3-pyridylmethyl)acetoacetate

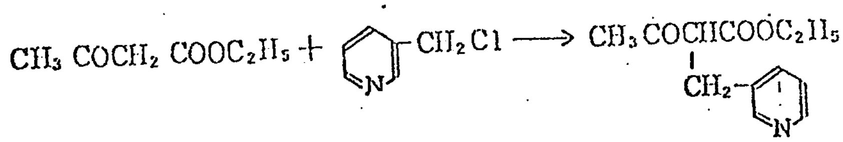

Under nitrogen, 8.4 g of metallic sodium was dissolved in 500 ml of anhydrous ethanol, and after adding 23.8 g of ethyl acetoacetate thereto and stirring the solution at room temperature for 1 hour, 30 g of 3-chloromethylpyridine hydrochloride was added to the solution followed by heating mildly for 20 hours. After filtering away the salt formed, the solvent was distilled off under reduced pressure, and the residue formed was purified by column chromatography using chloroform - ethyl acetate as the developing solvent to provide 12.0 g of the oily product.

NMR (in $CDCl_3$)

| | | |
|---|---|---|
| δ (ppm) ; | 1.18 | (t, 3H) |
| | 2.20 | (s, 3H) |
| | 3.13 | (d, 2H) |
| | 3.78 | (t, 1H) |
| | 4.13 | (q, 2H) |
| | 7.04~7.60 | (m, 2H) |
| | 8.40 | (m, 2H) |

Mass. (m/z) 221 ($M^+$)

Reference Examples 2 - 18

By following the same procedure as in Reference Example 1, the following compounds were obtained:

Reference Example 2

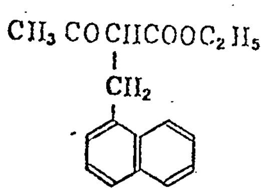

ethyl α-(1-naphthylmethyl)acetoacetate

NMR (in $CDCl_3$)

δ (ppm) ; 1.16 (t, 3H)

2.15 (s, 3H)

3.64 (d, 2H)

3.95 (t, 1H)

4.13 (q, 2H)

7.2~8.2 (m, 7H)

Mass (m/z) 270 ($M^+$), 227, 181

Reference Example 3

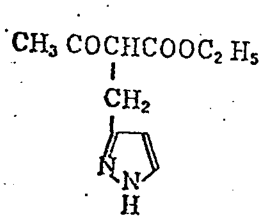

ethyl α-(3-pyrazolylmethyl)acetoacetate

NMR (in $CDCl_3$)

δ (ppm) ; 1.24 (t, 3H)

2.24 (s, 3H)

3.24 (d, 2H)

3.94 (t, 1H)

4.19 (q, 2H)

6.10 (d, 1H)

7.50 (d, 1H)

Mass (m/z) 210 ($M^+$), 167

Reference Example 4

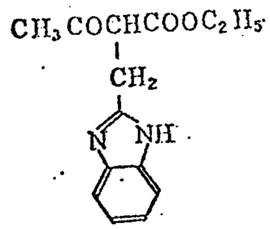

ethyl α-(2-benzimidazolylmethyl)-acetoacetate

Mass (m/z) 260 ($M^+$), 217

Reference Example 5

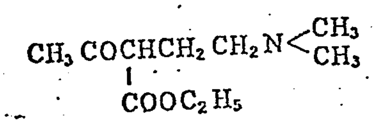

ethyl α-(2-N,N-dimethyl-aminoethyl)acetoacetate

NMR ($CDCl_3$)
δ (ppm); 1.28 (t, 3H)
2.08 (q, 2H)
2.1~2.4 (11H)
3.58 (t, 1H)
4.20 (q, 2H)

Mass (m/z) 201 ($M^+$), 156

Reference Example 6

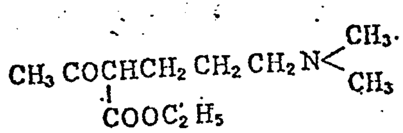

ethyl α-(3-N,N-dimethyl-aminopropyl)acetoacetate

NMR ($CDCl_3$)
δ (ppm); 1.28 (t, 3H)
1.4~1.6 (m, 2H)
1.7~2.0 (m, 2H)
2.1~2.5 (11H)
3.44 (t, 1H)
4.19 (q, 2H)

Mass (m/z) 215 ($M^+$), 170

Reference Example 7

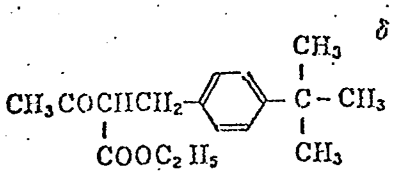

ethyl α-(p-tert-butylbenzyl)acetoacetate

NMR($CDCl_3$)
δ(ppm); 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00~7.36 (m, 4H)

IR(neat)
ν; 1735, 1710 $cm^{-1}$

Mass(m/z)
276 ($M^+$), 261 ($M^+$-15)
233 ($M^+$-43)

Reference Example 8

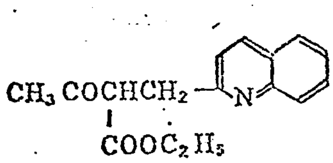

ethyl α-(2-quinolylmethyl)-acetoacetate

NMR($CDCl_3$)
δ(ppm); 1.26 (t, 3H)
2.45 (s, 3H)
3.50~3.67 (m, 2H)
4.20 (q, 2H)
4.54 (t, 1H)
7.20~8.08 (m, 6H)

IR(neat)
ν; 1730, 1705 $cm^{-1}$

Reference Example 9

Mass (m/z)
271 ($M^+$), 256 ($M^+$-15)
228 ($M^+$-43)

ethyl α-(p-chlorobenzyl)acetoacetate

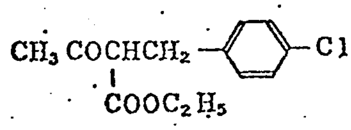

NMR ($CDCl_3$)
δ (ppm) ; 1.18 (t, 3H)
2.17 (a, 3H)
3.08 (d, 2H)
3.70 (t, 1H)
4.10 (q, 2H)
6.90~7.45 (m, 4H)

IR (neat)
ν ; 1735, 1710 $cm^{-1}$

Mass (m/z)
254 ($M^+$), 211 ($M^+$-43)

Reference Example 10

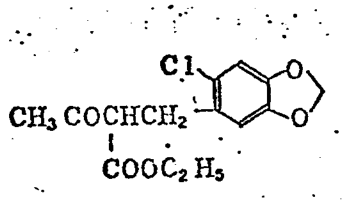

ethyl α-[(6-chloro-1,3-benzodioxole-5-yl)methyl]-acetoacetate

NMR ($CDCl_3$)
δ (ppm) ; 1.20 (t, 3H)
2.18 (s, 3H)
2.94~3.35 (m, 2H)
3.60~3.87 (m, 1H)
4.10 (q, 2H)
5.83 (s, 2H)
6.54~6.81 (m, 2H)

IR (neat)
ν ; 1735, 1710 $cm^{-1}$

Reference Example 11

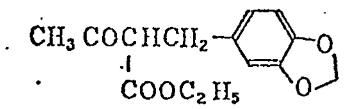

ethyl α-[(1,3-benzodioxole-5-yl)methyl]-acetoacetate

NMR($CDCl_3$)

δ(ppm); 1.20 (t, 3H)
2.16 (s, 3H)
3.05 (d, 2H)
3.68 (t, 1H)
4.10 (q, 2H)
5.82 (s, 2H)
6.58 (s, 3H)

IR(neat)

ν; 1730, 1710 $cm^{-1}$

Mass (m/z)

264 ($M^+$), 191 ($M^+$-73)

Reference Example 12

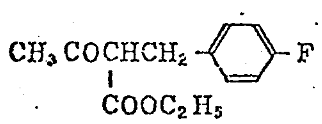

ethyl α-(p-fluorobenzyl)acetoacetate

Mass (m/z) 234 ($M^+$)

IR(neat) ν; 1700 $cm^{-1}$

NMR($CDCl_3$)

δ(ppm); 1.22 (t, 3H)
2.18 (s, 3H)
2.30 (s, 3H)
3.16 (d, 2H)
3.70 (dt, 1H)
4.14 (q, 2H)
7.04 (s, 4H)

Reference Example 13

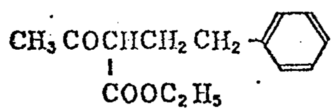

ethyl α-phenethyl-
acetoacetate

Mass (m/z) 235 ($M^+$+1)
NMR ($CDCl_3$)
δ (ppm) ; 1.25 (t, 3H)
2.10~2.75 (m, 4H)
2.20 (s, 3H)
3.35 (t, 1H)
4.20 (q, 2H)
7.15 (s, 5H)

Reference Example 14

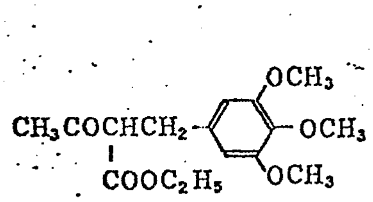

ethyl α-[(3,4,5-tri-
methoxy)benzyl]aceto-
acetate

Mass (m/z) 310 ($M^+$)
NMR ($CDCl_3$)
δ (ppm) ; 1.22 (t, 3H)
2.20 (s, 3H)
3.12 (d, 2H)
3.6~3.95 (m, 10H)
4.18 (q, 2H)
6.40 (s, 2H)

Reference Example 15

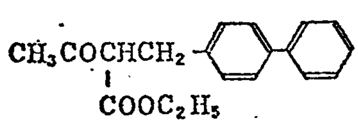

ethyl α-[(p-phenyl)-
benzyl]acetoacetate

Mass (m/z) 224 ($M^+$-COOEt+1)
IR (neat) ν ; 1710 $cm^{-1}$
NMR ($CDCl_3$)
δ (ppm) ; 1.22 (t, 3H)
2.20 (s, 3H)
3.20 (d, 2H)
3.78 (dt, 1H)
4.18 (q, 2H)
7.10~7.65 (m, 9H)

Reference Example 16

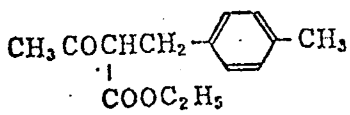

ethyl α-(p-methylbenzyl)acetoacetate

Mass (m/z) 234 ($M^+$)
IR (neat) ν ; 1700 $cm^{-1}$
NMR ($CDCl_3$)
δ (ppm) ; 1.22 (t, 3H)
2.18 (s, 3H)
2.30 (s, 3H)
3.16 (d, 2H)
3.70 (dt, 1H)
4.14 (q, 2H)
7.04 (s, 4H)

Reference Example 17

$CH_3COCH(CH_2)_5CH_3$
|
$COOC_2H_5$

ethyl α-n-hexylacetoacetate

Mass (m/z) 214 ($M^+$)
NMR ($CDCl_3$)
δ (ppm) ; 0.80~2.05 (m, 16H)
2.20 (s, 3H)
3.35 (t, 1H)
4.15 (q, 2H)

Reference Example 18

$CH_3COCH(CH_2)_7CH_3$
|
$COOC_2H_5$

ethyl α-n-octylacetoacetate

Mass (m/z) 242 ($M^+$)
NMR ($CDCl_3$)
δ (ppm) ; 0.80~2.00 (m, 20H)
2.20 (s, 3H)
3.35 (t, 1H)
4.20 (q, 2H)

Reference Example 19

Ethyl formacetate

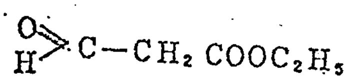

Under nitrogen, 0.23 g of chipped metallic sodium was dissolved in 10 ml of dry ether; while stirring vigorously, a solution of 0.88 g of ethyl acetate and 0.81 g of ethyl formate in 5 ml of dry ether was added dropwise to the mixture maintained at a temperature below -10°C for 3 hours, and the stirring was continued for 2 hours under these conditions. Thereafter, the mixture was stirred at room temperature for 1 day, and the solvent was distilled off under reduced pressure to provide the unpurified oily product.

Reference Example 20

By following the same procedure as in Reference Example 19, the following compounds were obtained.

(1) 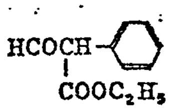

ethyl α-phenyl-formacetate

(2) 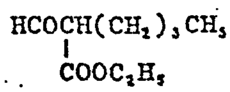

ethyl α-n-butyl-formacetate

(3) 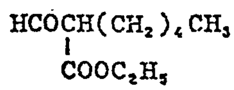

ethyl α-n-pentyl formacetate

Reference Examples 21 - 30.

By following the same procedure as in Reference Example 1, the following compounds were obtained:

Reference Example 21

$CH_3COCHCH_2CH_2CH_2N<^{C_2H_5}_{C_2H_5}$
$COOC_2H_5$

b.p. 110~114℃/1.8 mmHg
Mass (m/z) 243 ($M^+$)
198 ($M^+-45$)

ethyl ≪-(3-diethyl-aminopropyl)acetoacetate

Reference Example 22

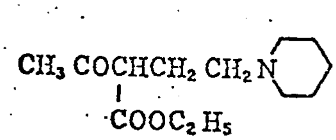

b.p. 125~130℃/2.0 mmHg
Mass (m/z) 241 ($M^+$)
196 ($M^+-45$)

ethyl ≪-(2-piperidino-ethyl)acetoacetate

Reference Example 23

$CH_3COCHCH_2CH_3$
$COOC_2H_5$

b.p. 110℃/25 mmHg
Mass (m/z) 159 ($M^++1$)

ethyl ≪-ethylacetoacetate

Reference Example 24

$CH_3COCHCH_2CH_2CH_3$
$\quad\quad\quad|$
$\quad\quad COOC_2H_5$

b.p. 125~130℃/25mmHg

Mass (m/z) 173 ($M^+$+1)

ethyl α-propyl-
acetoacetate

Reference Example 25

$CH_3COCHCH_2C{\equiv}CH$
$\quad\quad\quad|$
$\quad\quad COOC_2H_5$

b.p. 135~140℃/25mmHg

Mass (m/z) 169 ($M^+$+1)

ethyl α-(2-propinyl)-
acetoacetate

Reference Example 26

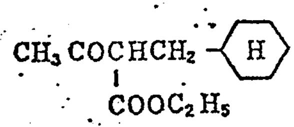

Mass (m/z) 227 ($M^+$+1)

NMR ($CDCl_3$ )

δ (ppm) ; 0.8~1.90 (m, 13H)
1.25 (t, 3H)
2.30 (s, 3H)
3.45 (t, 1H)
4.15 (q, 2H)

ethyl α-cyclohexyl-
methylacetoacetate

Reference Example 27

$CH_3COCH(CH_2)_3CH_3$
$\quad\quad\quad|$
$\quad\quad COOC_2H_5$

Mass (m/z) 187 ($M^+$+1)

NMR ($CDCl_3$ )

δ (ppm) ; 0.75~2.05 (m, 9H)
1.25 (t, 3H)
3.35 (t, 1H)
4.15 (q, 2H)

ethyl α-n-butylaceto-
acetate

Reference Example 28

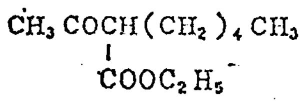

ethyl α-n-pentyl-
acetoacetate

Mass (m/z) 201 ($M^+$+1)
NMR ($CDCl_3$)
δ (ppm) ; 0.70~2.05 (m, 11H)
1.25 (t, 3H)
3.35 (t, 1H)
4.15 (q, 2H)

Reference Example 29

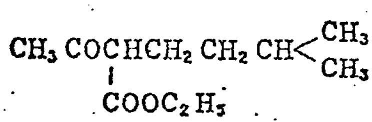

ethyl α-[(3-methyl)-
butyl] acetoacetate

Mass (m/z) 201 ($M^+$+1)
NMR ($CDCl_3$)
δ (ppm) ; 0.90 (d, 6H)
1.05~2.05 (m, 5H)
1.25 (t, 3H)
3.30 (t, 1H)
4.15 (q, 2H)

Reference Example 30

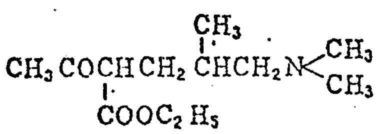

ethyl α-[3-(N,N-dimethyl-
amino)-2-methylpropyl]-
acetoacetate

b.p. 75~78℃/0.4 mmHg
Mass (m/z) 229 ($M^+$)

Reference Example 31

(1)

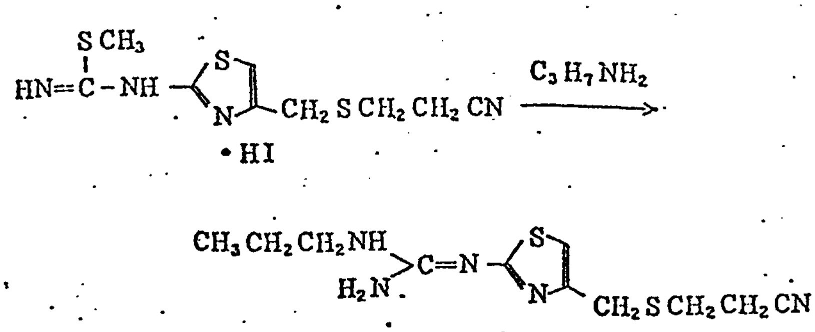


8.75 g of 3-[[[2-(8-methylisothioureido)thiazole-4-yl]methyl]thio]propionitrile hydroiodide and 12.9 g of n-propylamine were dissolved in 200 ml of ethanol, and the solution was refluxed under heating for 48 hours. The solvent was distilled away, and the residue formed was purified by column chromatography using a mixture of chloroform and methanol as the developing solvent to provide 6.0 g of 3-(2-n-propylguanidinothiazol-4-ylmethylthio)propionitrile

NMR ($CDCl_3$)

δ (ppm) ; 1.03 (t, 3H)

1.68 (m, 2H)

2.4–2.9 (m, 4H)

3.20 (t, 2H)

3.72 (s, 2H)

6.44 (s, 1H)

Mass (m/z) 283 ($M^+$)

(2) 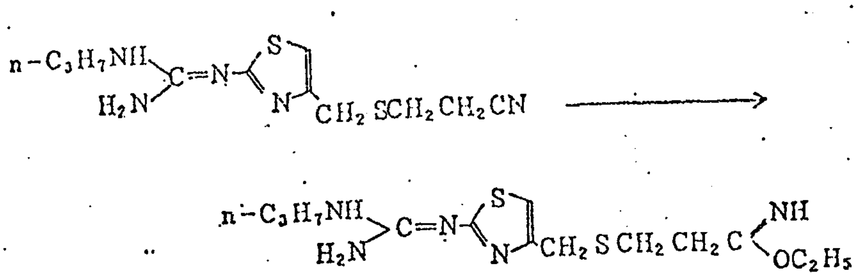

6.0 g of the nitrile compound obtained as in (1) above was dissolved in 100 ml of dry chloroform and after adding 20 ml of dry ethanol thereto and cooling the solution below 10°C and passing therethrough dry hydrogen chloride gas for 3 hours, the solution was allowed to stand at 0-4°C for 48 hours. Then, the solvents were distilled off and the residue was poured into a cooled aqueous solution of potassium carbonate for alkalifying, and the mixture was extracted with chloroform. The chloroform layer was washed with water and dried over anhydrous magnesium sulfate, and the solvent was distilled off under reduced pressure to provide 6.0 g of the oily product.

(3) 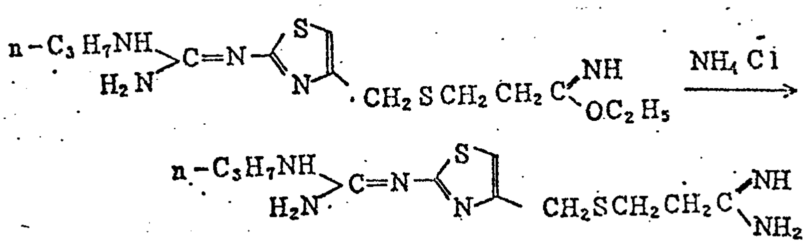


A mixture of 6.0 g of the imidate compound obtained as at (2) above, and 1.0 g of ammonium chloride in 70 ml of methanol was stirred at room temperature for 1 hour, and the solvent was distilled off under reduced pressure. The residue was treated with hydrochloric acid in ethanol, and was recrystallized with acetonitrile - ethanol to provide 5.8 g of the amidine hydrochloride having a melting point of 183 - 184.5°C.

Reference Example 32

4-(m-piperidinomethylphenoxy) butaneamidine hydrochloride

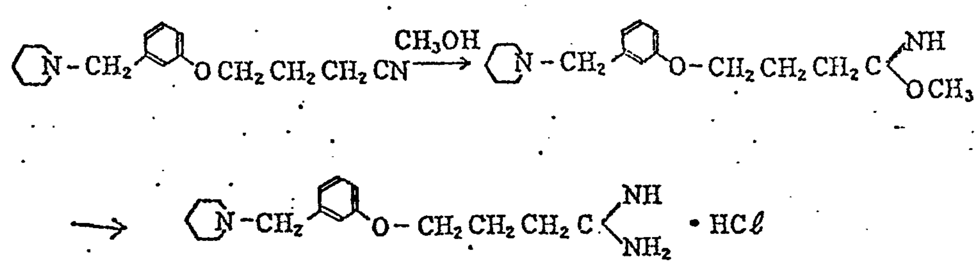


To 200 ml of dry chloroform were added 20.0 g (77.4 ml) of 4-(m-piperidinomethylphenoxy)butanenitrile and 15.7 ml (387 mmol) of dry methanol, and the solution obtained was cooled by ice-water bath. Through the solution was passed hydrogen chloride gas at 10°C. After confirming the disappearance of the starting material in 8 hours, the reaction was stopped, and the solvent was distilled off under reduced pressure. The residue was dissolved in chloroform, and the solution was added to saturated aqueous potassium carbonate solution and stirred. The organic solvent layer was separated, and the aqueous layer was extracted with chlorform. The organic layer and the chloroform solution were combined, and the combined solution was dried over anhydrous magnesium sulfate. The solvent was distilled away to provide oily crude methyl 4-(m-piperidinomethylphenoxy)butaneimidate (21.1 g). The product was dissolved in 400 ml of dry methanol, and 3.5 g (65.4 mmol) of ammonium chloride was added to the obtained solution at room temperature. The solution became homogeneous in 10 minutes, and completion of the reaction was checked by thin layer chromatography. The imidate disappeared after stirring the solution for 3 hours (checked by chromatography). A small amount of precipitate was filtered away and the solution was concentrated to precipitate crystals. The precipitated crystals were collected by filtration to provide 12.3 g of colourless needle crystals.

The filtrate was concentrated to dryness. By recrystallization from ethyl acetate-ethanol, 5.6 g of product was obtained (18.0 mmol, 23%).

m.p. 151.0 - 152.0 °C (ethanol-ethyl acetate)

Mass. (m/e) 275 ($M^+$ - HCl)

Elemental analysis for $C_{16}H_{25}N_3O$ HCl

| | C | H | N | Cl |
|---|---|---|---|---|
| Calculated | 61.62 | 8.40 | 13.47 | 11.37 |
| Found | 61.47 | 8.51 | 13.52 | 11.41 |

Reference Example 33

Ethyl α-(3-pyridylmethyl)acetoacetate

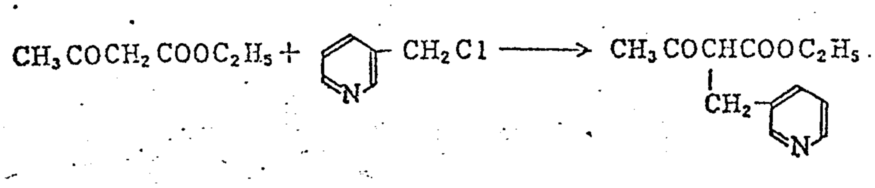

In 500 ml of anhydrous ethanol was dissolved 8.4 g of metallic sodium and after adding thereto 23.8 g of acetoacetate, the mixture was stirred for 1 hour at room temperature. Then, 30 g of 3-chloromethylpyridine hydrochloride was added to the mixture, and the mixture obtained was refluxed under mild heating. The salt formed was filtered away and the solvent was then distilled off under reduced pressure. The residue was purified by column chromatography using chloroform - ethyl acetate as a developing solvent to provide 12.0 g of oily product.

NMR ($CDCl_3$)

δ (ppm) ; 1.18 (3H, t)

2.20 (3H, s)

3.13 (2H, d)

3.78 (1H, t)

4.13 (2H, q)

7.04~7.60 (2H, m)

8.40 (2H, m)

Mass (m/e) 221 ($M^+$)

Reference Examples 34-38

By following the same procedure in Reference EXample 33, the following compounds were obtained:

Reference Example 34

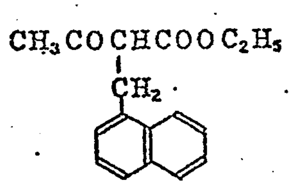

ethyl α-(1-naphythyl-methyl)acetoacetate

NMR ($CDCl_3$)

δ (ppm) ; 1.16 (3H, t)

2.15 (3H, s)

3.64 (2H, d)

3.95 (1H, t)

4.13 (2H, q)

7.2~8.2 (7H, m)

Mass (m/e) 270 ($M^+$), 227, 181

0173377

Reference Example 35

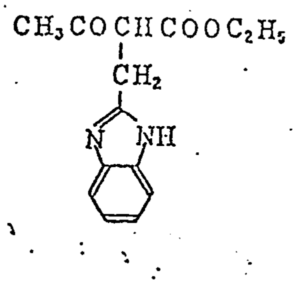

ethyl α-(2-benzimidazolyl-methyl)acetoacetate

Mass (m/e) 260 ($M^+$), 217

Reference Example 36

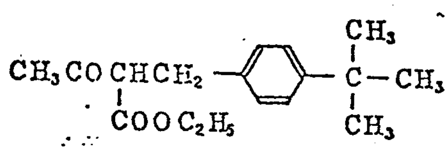

ethyl α-(p-tert-butylbenzyl)-acetoacetate

NMR ($CDCl_3$ 中)

δ (ppm) ; 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00～7.36 (m, 4H)

IR (neat)
ν ; 1735, 1710 $cm^{-1}$

Mass (m/e)
276 ($M^+$), 261 ($M^+-15$)
233 ($M^+-43$)

0173377



Reference
Example 37

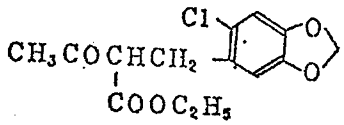

NMR ( $CDCl_3$ )
δ(ppm) ; 1.20 ( t, 3H)
2.18 ( s, 3H)
2.94~3.35 (m, 2H)
3.60~3.87 (m, 1H)
4.10 ( q, 2H)
5.83 ( s, 2H)
6.54~6.81 (m, 2H)

ethyl α- (6-chloro-1,3-benzodioxole-5-yl)methyl - acetoacetate.

IR ( neat)
ν ; 1735, 1710 $cm^{-1}$
Mass (m/e)
298 ($M^+$), 255 ($M^+ - 43$)

Reference
Example 38

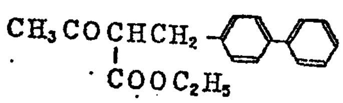

NMR ( $CDCl_3$ )
δ(ppm) ; 1.22 ( t, 3H)
2.20 ( s, 3H)
3.20 ( d, 2H)
3.78 ( dt, 1H)
4.18 ( q, 2H)
7.10~7.65 (m, 9H)

ethyl α-(p-phenylbenzyl)-acetoacetate

IR ( neat)
ν ; 1710 $cm^{-1}$
Mass (m/e)
224 ($M^+ - COOC_2H_5 + 1$)

Example 1

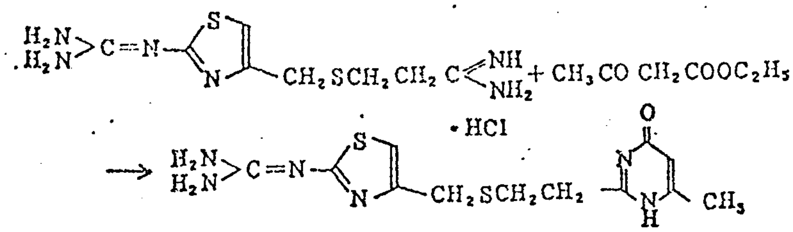

0.916 g of sodium methoxide (MeONa) was dissolved in 10 ml of methanol, and to the solution formed was added a solution of 5 g of 3-[[[2-[(diaminomethylene)amino]-4-thiazolyl]methyl]-thio]propionamidine hydrochloride in 50 ml of methanol followed by stirrring, and after adding 2.2 g of ethyl acetoacetate and stirring the mixture at room temperature for 20 hours, the salt formed was filtered away and the solvents were distilled off under reduced pressure. The residue was purified by column chromatography using chloroform - methanol as the developing solvent, and was recrystallized from ethanol to provide 2-[2-[[[2-[(diaminomethylene)amino]-4-thiazolyl]methyl]thio]ethyl]--6-methyl-4-(lH)-pyrimidone (1.3 g) having a melting point of 176 - 177°C.

Anal. ($C_{12}H_{16}N_6OS_2$)

| | | C | H | N |
|---|---|---|---|---|
| Calc. | (%) | 44.43 | 4.97 | 25.90 |
| Found | (%) | 44.39 | 4.97 | 25.86 |

Examples 2 - 38

By following the same procedure as in Example 1, the following compounds were obtained:

Example 2

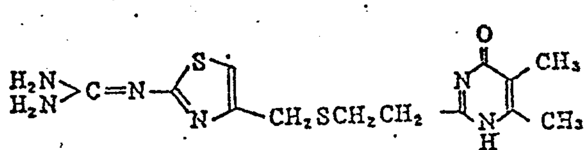

2-[2-[[[2-[(diaminomethylene)amino]-4-thiazolyl]methyl]thio]-ethyl]-5,6-dimethyl-4(1H)-pyrimidone

m.p. 199～200℃

Anal. ( $C_{13}H_{18}N_6OS_2$)

| | C | H | N |
|---|---|---|---|
| Calc. | 46.14 | 5.36 | 24.83 |
| Found | 46.13 | 5.35 | 24.62 |

Example 3

5-benzyl-2-[2-[[[2-[(diaminomethylene)amino]-4-thiazolyl]-methyl]thio]ethyl]-6-methyl-4(1H)-pyrimidone

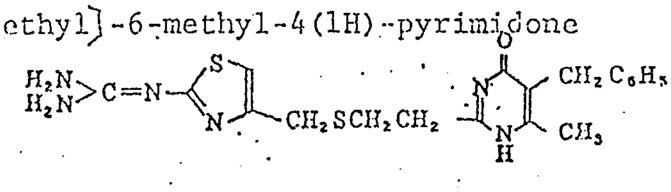

m.p. 149 ~ 150 ℃

Anal. ( $C_{19}H_{22}N_6OS_2$ )

| | | C | H | N |
|---|---|---|---|---|
| Calc. | (%) | 55.05 | 5.35 | 20.27 |
| Found | (%) | 55.01 | 5.39 | 19.79 |

Example 4

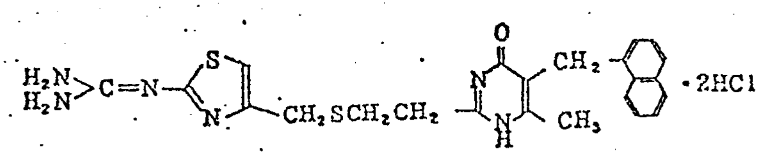

2-[2-[[[2-[(diaminomethylene)amino]-4-thiazolyl]methyl]thio]-ethyl]-6-methyl-5-(1-naphthylmethyl)-4(1H)-pyrimidone dihydrochloride

m.p. 202 ~ 207℃

Anal. ( $C_{23}H_{26}N_6OS_2Cl_2$ )

| | | C | H | N |
|---|---|---|---|---|
| Calc. | (%) | 51.39 | 4.88 | 15.63 |
| Found | (%) | 51.45 | 4.78 | 15.67 |

Example 5

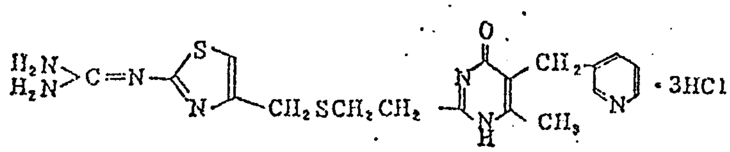

2-[2-[[[2-[(diaminomethylene)amino]-4-thiazolyl]methyl]thio]-ethyl]-6-methyl-5-(3-pyridylmethyl)-4(1H)-pyrimidone trihydrochloride

m.p. 184 ~ 186 °C

Anal. ($C_{18}H_{24}N_7OS_2Cl_3$)

| | C | H | N |
|---|---|---|---|
| Cal | 41.19 | 4.61 | 18.68 |
| Found | 41.28 | 4.65 | 18.62 |

Example 6

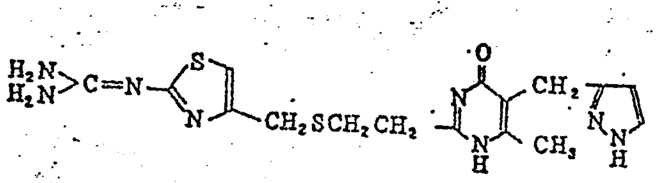

2-[2-[[[2-[(diaminomethylene)amino]-4-thiazolyl]methyl]thio]-ethyl]-6-methyl-5-[(3-pyrazolyl)methyl]-4(1H)-pyrimidone

m.p. 209 ~ 211 °C

Anal. ($C_{16}H_{20}N_8OS_2$)

| | | C | H | N |
|---|---|---|---|---|
| Calc. | (%) | 47.51 | 4.98 | 27.70 |
| Found | (%) | 47.13 | 4.95 | 27.48 |

Example 7

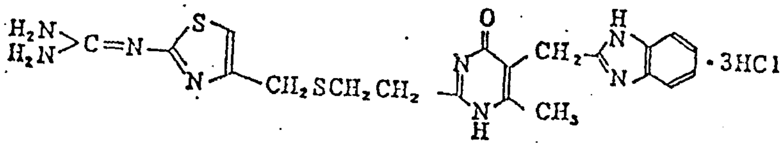

5-(2-benzimidazolylmethyl)-2-[2-[[[2-[(diaminomethylene)-amino]-4-thiazolyl]methyl]thio]ethyl]-6-methyl-4(1H)-pyrimidone trihydrochloride

m.p. 226 ~ 229 ℃

Anal. ($C_{20}H_{25}N_8OS_2Cl_3$)

| | | C | H | N |
|---|---|---|---|---|
| Calc. | (%) | 42.60 | 4.47 | 19.87 |
| Found | (%) | 42.69 | 4.55 | 19.65 |

Example 8

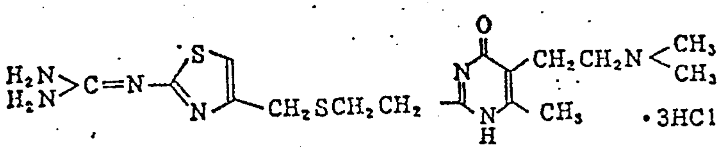

2-[2-[[[2-[(diaminomethylene)amino]-4-thiazolyl]methyl]thio]-ethyl]-5-(2-dimethylaminoethyl)-6-methyl-4(1H)-pyrimidone trihydrochloride

m.p. 164 ~ 167 ℃

Anal. ($C_{16}H_{23}N_7OS_2Cl_3$)

| | | C | H | N |
|---|---|---|---|---|
| Calc. | (%) | 38.06 | 5.59 | 19.42 |
| Found | (%) | 38.16 | 5.59 | 19.17 |

Example 9

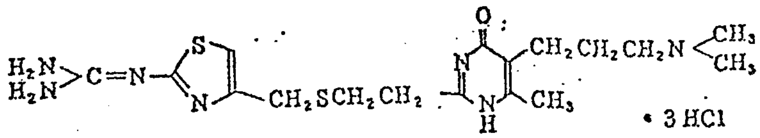

2-[2-[[[2-[(diaminomethylene)amino]-4-thiazolyl]methyl] thio]-ethyl] -5-(3-dimethylaminopropyl)-6-methyl-4(1H)-pyrimidone tri-hydrochloride

m.p. 175 ~ 177°C

Anal. ($C_{17}H_{30}N_7OS_2Cl_3 \cdot \frac{1}{2} H_2O$)

| | | C | H | N |
|---|---|---|---|---|
| Calc. | (%) | 38.67 | 5.92 | 18.57 |
| Found | (%) | 38.38 | 5.90 | 18.46 |

Example 10

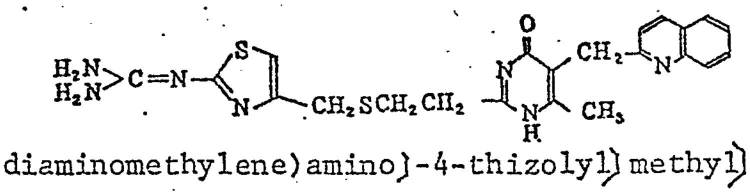

2-[2-[[[2-[(diaminomethylene)amino]-4-thizolyl] methyl] thio] -ethyl] -6-methyl-5-(2-quinolylmethyl)-4(1H)-pyrimidone

m.p. 218 ~ 220°C

Anal. ($C_{22}H_{23}N_7OS_2$)

| | | C | H | N | S |
|---|---|---|---|---|---|
| Calc. | (%) | 56.75 | 4.98 | 21.06 | 13.77 |
| Found | (%) | 56.55 | 5.00 | 20.84 | 13.94 |

Example 11

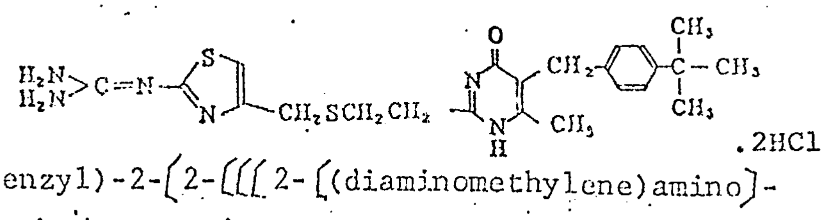

.2HCl

5-(p-tert-butylbenzyl)-2-[2-[[[2-[(diaminomethylene)amino]-4-thiazolyl]methyl]thio]ethyl]-6-methyl-4(1H)-pyrimidone dihydrochloride

m.p. 190.5 ~ 193.0°C

Anal. ($C_{23}H_{32}N_6OS_2Cl_2$)

| | | C | H | N |
|---|---|---|---|---|
| Calc. | (%) | 50.82 | 5.93 | 15.46 |
| Found | (%) | 50.51 | 5.81 | 15.38 |

Example 12

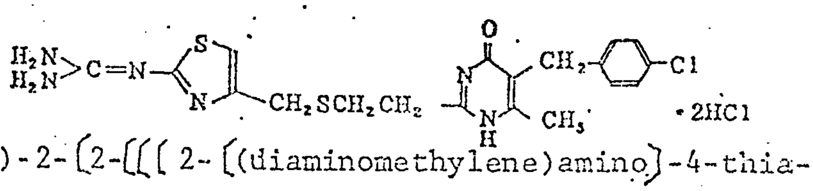

•2HCl

5-(p-chlorobenzyl)-2-[2-[[[2-[(diaminomethylene)amino]-4-thiazolyl]methyl]thio]ethyl]-6-methyl-4(1H)-pyrimidone dihydrochloride

m.p. 194.5 ~ 198.0°C

Anal. ($C_{19}H_{23}N_6OS_2Cl_3$)

| | | C | H | N |
|---|---|---|---|---|
| Calc. | (%) | 43.73 | 4.44 | 16.10 |
| Found | (%) | 43.50 | 4.16 | 15.90 |

Example 13

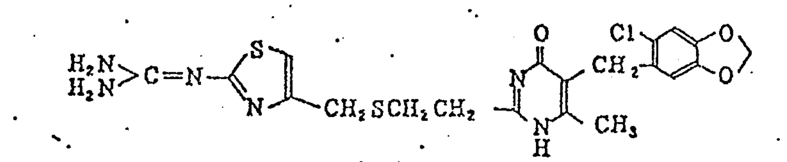

5-[(6-chloro-1,3-benzodioxole-5-yl)methyl]-2-[2-[[[2-[(diaminomethylene)amino]-4-thiazolyl]methyl]thio]ethyl]-6-methyl-4(1H)-pyrimidone

m.p. 201.5 ~ 202.5 ℃

Anal. ($C_{20}H_{21}ClN_6O_3S_2$)

| | | C | H | N |
|---|---|---|---|---|
| Calc. | (%) | 48.73 | 4.29 | 17.05 |
| Found | (%) | 48.65 | 4.30 | 17.08 |

Example 14

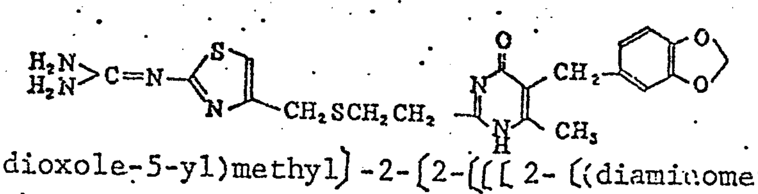

5-[(1,3-benzodioxole-5-yl)methyl]-2-[2-[[[2-[(diaminomethylene)-amino]-4-thiazolyl]methyl]thio]ethyl]-6-methyl-4(1H)-pyrimidone

m.p. 148.5 ~ 150.5 ℃

Anal. ($C_{19}H_{22}N_6O_3S_2$)

| | | C | H | N |
|---|---|---|---|---|
| Calc. | (%) | 52.39 | 4.84 | 18.33 |
| Found | (%) | 52.36 | 4.63 | 18.31 |

Example 15

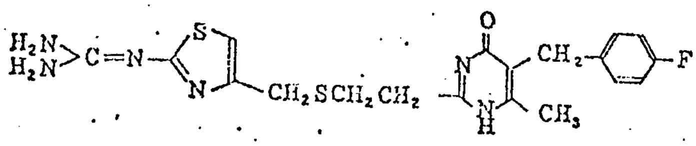

2-{2-[[[2-[(diaminomethylene)amino]-4-thiazolyl]methyl]thio]-ethyl}-5-(p-fluorobenzyl)-6-methyl-4(1H)-pyrimidone

m.p. 139 ~ 141 ℃ (recrystallized from ethanol-ethylacetoacetate)

Mass. (m/z) 432 ($M^+$)

Anal. ($C_{19}H_{21}N_6OS_2F$)

| | | C | H | N | S |
|---|---|---|---|---|---|
| Calc. | (%) | 52.76 | 4.89 | 19.43 | 14.82 |
| Found | (%) | 52.52 | 5.02 | 19.20 | 14.79 |

Example 16

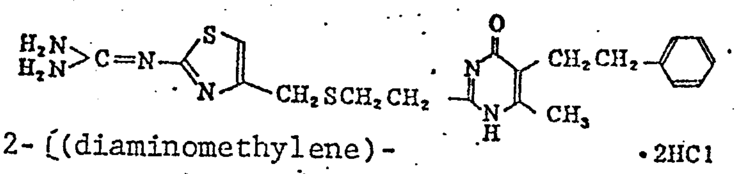

2-{2-[[[2-[(diaminomethylene)-amino]-4-thiazolyl]methyl]thio]ethyl}-6-methyl-5-phenethyl-4(1H)-pyrimidone di-hydrochloride

•2HCl

m.p. 205 ~ 208 ℃ (methanol)

Mass. (m/z) 428 ($M^+$)

Anal. ($C_{20}H_{24}N_6OS_2 \cdot 2HCl$)

| | | C | H | N | S | Cl |
|---|---|---|---|---|---|---|
| Calc. | (%) | 47.90 | 5.23 | 16.70 | 12.79 | 14.14 |
| Found | (%) | 47.73 | 5.20 | 16.86 | 12.97 | 13.99 |

Example 17

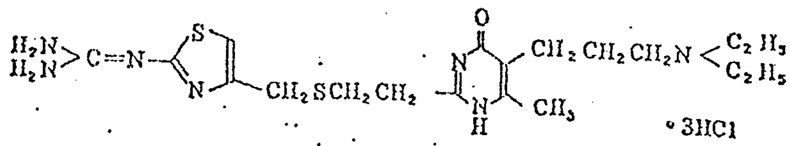

2-[2-[[[2-[(diaminomethylene)amino]-4-thiazolyl]methyl]-thio]ethyl]-5-(3-diethylaminopropyl)-6-methyl-4(1H)-pyrimidone tri-hydrochloride

m.p. 179 ~ 181 °C (ethanol)

Anal. ($C_{19}H_{34}N_7OS_2Cl_3$)

| | | C | H | N | S | Cl |
|---|---|---|---|---|---|---|
| Calc. | (%) | 41.72 | 6.26 | 17.92 | 11.72 | 19.44 |
| Found | (%) | 41.68 | 6.47 | 17.82 | 11.63 | 19.41 |

Example 18

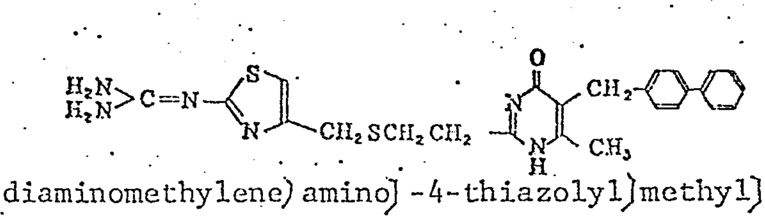

2-[2-[[[2-[(diaminomethylene)amino]-4-thiazolyl]methyl]thio]-ethyl]-6-methyl-5-(p-phenylbenzyl)-4(1H)-pyrimidone

m.p. 129.0 ~ 130.5 °C (methanol)

Mass. (m/z) 491 ($M^+$+1)

Anal. ($C_{25}H_{26}N_6OS_2$)

| | | C | H | N | S |
|---|---|---|---|---|---|
| Calc. | (%) | 61.20 | 5.34 | 17.13 | 13.07 |
| Found | (%) | 61.17 | 5.20 | 17.12 | 13.00 |

Example 19

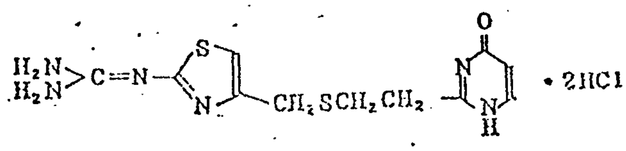

2-[2-[[[2-[(diaminomethylene)amino]-4-thiazolyl]methyl]thio]-ethyl]-4(1H)pyrimidone di-hydrochloride

m.p. 182～185 ℃ (methanol- chloroform)

Mass. (m/z) 310 ($M^+$)

Anal. ($C_{11}H_{14}N_6OS_2 \cdot 2HCl \cdot \frac{1}{2}H_2O$)

| | | C | H | N | S | Cl |
|---|---|---|---|---|---|---|
| Calc. | (%) | 33.68 | 4.37 | 21.42 | 16.34 | 18.07 |
| Found | (%) | 33.69 | 4.32 | 21.37 | 16.50 | 18.10 |

Example 20

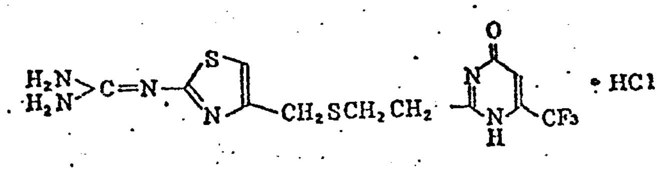

2-[2-[[[2-[(diaminomethylene)amino]-4-thiazolyl]methyl]thio]-ethyl]-6-trifluoromethyl-4(1H)-pyrimidone hydrochloride

m.p. 210～213 ℃ (methanol)

Mass. (m/z) 378 ($M^+$)

Anal. ($C_{12}H_{13}N_6OS_2F_3 \cdot HCl$)

| | | C | H | N | S | Cl |
|---|---|---|---|---|---|---|
| Calc. | (%) | 34.74 | 3.40 | 20.26 | 15.46 | 8.55 |
| Found | (%) | 34.64 | 3.17 | 20.13 | 15.53 | 8.76 |

Example 21

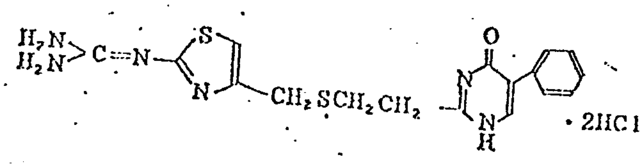

m.p. 175 ~ 179℃ (methanol)

2-[2-[[[2-[(diaminomethylene)amino]-4-thiazolyl]methyl]thio]-ethyl]-5-phenyl-4(1H)-pyrimidone di-hydrochloride

Mass. (m/z) 386 ($M^+$)

Anal. ($C_{17}H_{18}N_6OS_2 \cdot 2HCl \cdot H_2O$)

| | | C | H | N | S |
|---|---|---|---|---|---|
| Calc. | (%) | 42.77 | 4.64 | 17.60 | 13.43 |
| Found | (%) | 42.78 | 4.36 | 17.57 | 13.11 |

Example 22

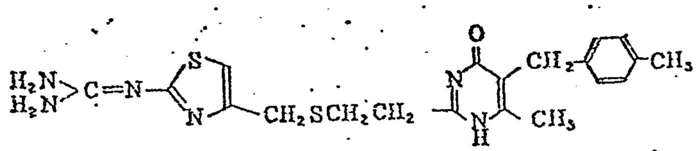

2-[2-[[[2-[(diaminomethylene)amino]-4-thiazolyl]methyl]thio]-ethyl]-6-methyl-5-(p-methylbenzyl)-4(1H)-pyrimidone

m.p. 133 ~ 135℃ (ethanol)

(2 HCl salt: m.p. 177 ~ 181℃ (ethanol))

Mass. (m/z) 428 ($M^+$)

Anal. ($C_{20}H_{24}N_6OS_2 \cdot 2HCl \cdot H_2O$)

| | | C | H | N | S | Cl |
|---|---|---|---|---|---|---|
| Calc. | (%) | 46.24 | 5.43 | 16.18 | 12.34 | 13.65 |
| Found | (%) | 46.44 | 5.13 | 16.30 | 12.58 | 13.64 |

Example 23

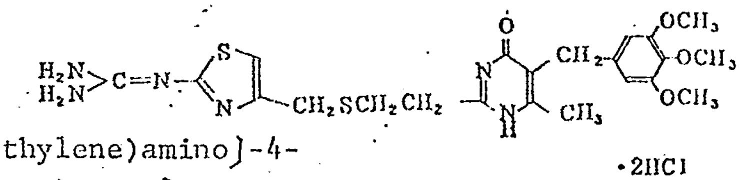

2-[2-[[[2-(diaminomethylene)amino]-4-thiazolyl]methyl]thio]ethyl]-5-(3,4,5-trimethoxybenzyl)-6-methyl-4(1H)-pyrimidone dihydrochloride

m.p. 168～171°C (methanol-ether)

Mass. (m/z) 504 ($M^+$)

Anal. ($C_{22}H_{28}N_6O_4S_2 \cdot 2HCl$)

| | | C | H | N | S | Cl |
|---|---|---|---|---|---|---|
| Calc. | (%) | 45.75 | 5.24 | 14.55 | 11.10 | 12.28 |
| Found | (%) | 45.46 | 5.29 | 14.70 | 10.94 | 12.42 |

Example 24

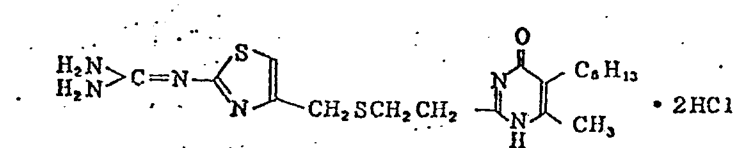

2-(2-(([2-[(diaminomethylene)amino]-4-thiazolyl]methyl]thio]-ethyl]-5-n-hexyl-6-methyl-4(1H)-pyrimidone di-hydrochloride

m.p. 166～169°C (ethanol-ethyl acetate)

Mass. (m/z) 408 ($M^+$)

Anal. ($C_{18}H_{28}N_6OS_2 \cdot 2HCl$)

| | | C | H | N | S | Cl |
|---|---|---|---|---|---|---|
| Calc. | (%) | 44.90 | 6.28 | 17.45 | 13.32 | 14.73 |
| Found | (%) | 44.80 | 6.29 | 17.47 | 13.51 | 14.64 |

Example 25

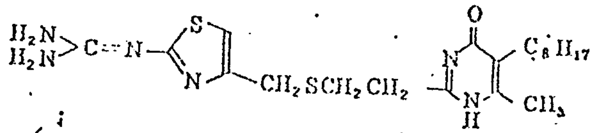

• 2HCl

2-(2-[[[2-[(diaminomethylene)amino]-4-thiazolyl]methyl]thio]-ethyl]-6-methyl-5-n-octyl-4(1H)-pyrimidone di-hydrochloride

m.p. 165～169 ℃ (ethanol-ethylacetate)

Mass. (m/z) 436($M^+$)

Anal. ($C_{20}H_{32}N_6OS_2 \cdot 2HCl$)

| | | C | H | N | S | Cl |
|---|---|---|---|---|---|---|
| Calc. | (%) | 47.14 | 6.73 | 16.49 | 12.58 | 13.92 |
| Found | (%) | 46.89 | 6.87 | 16.40 | 12.60 | 13.79 |

Example 26

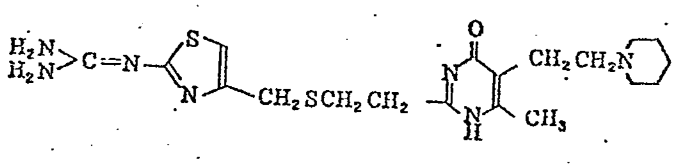

m.p. 156～159 ℃ (ethanol) .3HCL

Anal. ($C_{19}H_{32}N_7OS_2Cl_3 \cdot \frac{1}{2} H_2O$)

| | | C | H | N |
|---|---|---|---|---|
| Calc. | (%) | 41.19 | 6.00 | 17.70 |
| Found | (%) | 41.04 | 6.15 | 17.80 |

2-[2-[[[2-[(diaminomethylene)amino]-4-thiazolyl]methyl]-thio]ethyl]-6-methyl-5-(2-piperidinoethyl)-4(1H)-pyrimidone tri-hydrochloride

Example 27

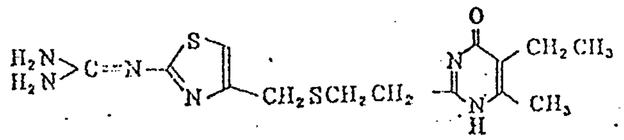

2-[2-[[[2-[(diaminomethylene)amino]-4-thiazolyl]methyl]thio]-ethyl]-5-ethyl-6-methyl-4(1H)-pyrimidone

m.p. 182～183 ℃ (ethanol-ethylacetate)

Anal. ($C_{14}H_{20}N_6OS_2$).

| | | C | H | N |
|---|---|---|---|---|
| Calc. | (%) | 47.71 | 5.72 | 23.84 |
| Found | (%) | 47.54 | 5.62 | 23.83 |

Example 28

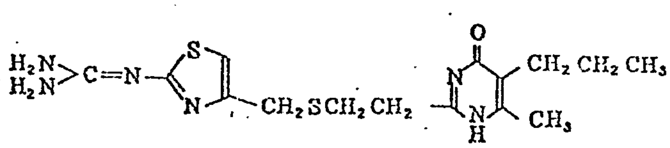

2-[2-[[[2-[(diaminomethylene)amino]-4-thiazolyl]methyl]thio]-ethyl]-6-methyl-5-propyl-4(1H)-pyrimidone

m.p. 218～219 ℃ (ethanol-ethylacetate)

Anal. ($C_{15}H_{22}N_6OS_2$).

| | | C | H | N |
|---|---|---|---|---|
| Calc. | (%) | 49.16 | 6.05 | 22.93 |
| Found | (%) | 48.86 | 6.06 | 23.00 |

Example 29

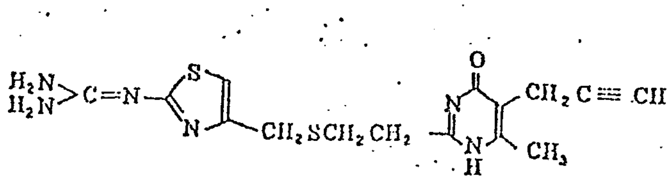

2-〔2-{[[2-〔(diaminomethylene)amino〕-4-thiazolyl]methyl]thio〕-ethyl〕-6-methyl-5-(2-propinyl)-4(1H)-pyrimidone

m.p. 208～210℃ (ethanol-ethylacetate)

Anal. ($C_{15}H_{18}N_6OS_2 \cdot \frac{1}{2}H_2O$)

| | | C | H | N |
|---|---|---|---|---|
| Calc. | (%) | 48.50 | 5.16 | 22.62 |
| Found | (%) | 48.65 | 4.94 | 22.46 |

Example 30

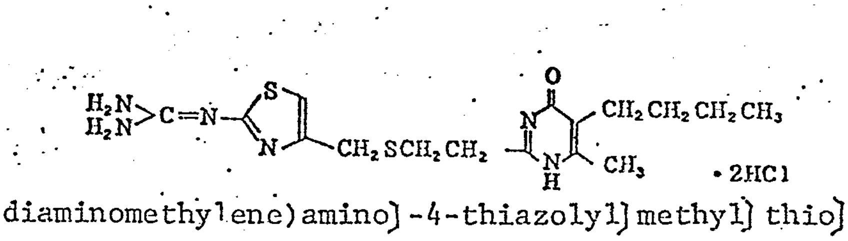

·2HCl

2-〔2-{[[2-〔(diaminomethylene)amino〕-4-thiazolyl]methyl] thio〕-ethyl〕-5-n-butyl-6-methyl-4(1H)-pyrimidone di-hydrochloride

m.p. 163～165℃ (ethanol-ethylacetate)

Mass. (m/z) 380 ($M^+$)

Anal. ($C_{16}H_{24}N_6OS_2 \cdot 2HCl \cdot \frac{1}{2}H_2O$)

| | | C | H | N | S | Cl |
|---|---|---|---|---|---|---|
| Calc. | (%) | 41.56 | 5.88 | 18.17 | 13.87 | 15.33 |
| Found | (%) | 41.77 | 5.91 | 18.34 | 13.96 | 15.36 |

24.07.85



Example 31

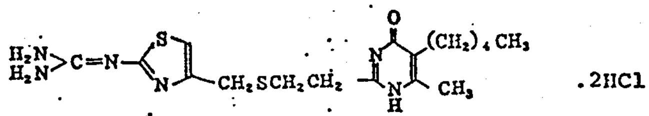

2-{2-[[[2-[(diaminomethylene)amino]-4-thiazolyl]methyl] thio]-ethyl}-6-methyl-5-n-pentyl-4(1H)-pyrimidone di-hydrochloride

m.p. 149～151℃ (ethanol-ethylacetate)

Mass. (m/z) 394 ($M^+$).

Anal. ($C_{17}H_{26}N_6OS_2 \cdot 2HCl \cdot \frac{1}{2}H_2O$)

| | | C | H | N | S |
|---|---|---|---|---|---|
| Calc. | (%) | 42.85 | 6.13 | 17.64 | 13.46 |
| Found | (%) | 42.82 | 6.09 | 17.60 | 13.19 |

Example 32

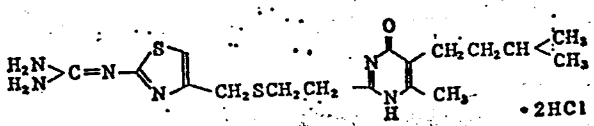

2-{2-[[[2-[(diaminomethylene)amino]-4-thiazolyl]methyl] thio]-ethyl}-6-methyl-5-(3-methylbutyl)-4(1H)-pyrimidone di-hydrochloride

m.p. 179～180.5℃ (ethanol-ethylacetate)

Mass. (m/z) 394 ($M^+$)

Anal. ($C_{17}H_{26}N_6OS_2 \cdot 2HCl \cdot \frac{1}{2}H_2O$)

| | | C | H | N | S | Cl |
|---|---|---|---|---|---|---|
| Calc. | (%) | 42.85 | 6.13 | 17.64 | 13.46 | 14.88 |
| Found | (%) | 43.06 | 5.89 | 18.01 | 13.62 | 15.03 |

Example 33

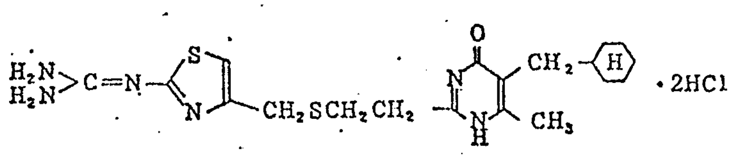

2-[2-[[[2-[(diaminomethylene)amino]-4-thiazolyl]methyl]thio]-ethyl]-5-cyclohexylmethyl-6-methyl-4(1H)-pyrimidone di-hydrochloride

m.p. 191～193℃ (methanol - iso-propanol)

Mass. (m/z) 420 ($M^+$)

Anal. ($C_{19}H_{28}N_6OS_2 \cdot 2HCl$)

| | | C | H | N | S | Cl |
|---|---|---|---|---|---|---|
| Calc. | (%) | 46.24 | 6.13 | 17.03 | 12.99 | 14.37 |
| Found | (%) | 46.11 | 6.32 | 17.09 | 12.79 | 14.23 |

Example 34

2-[2-[[[2-[(diaminomethylene)amino]-4-thiazolyl]methyl]thio]-ethyl]-6-methyl-5-(2-methyl-3-dimehtylaminopropyl)-4(1H)-pyrimidone tri-hydrochloride

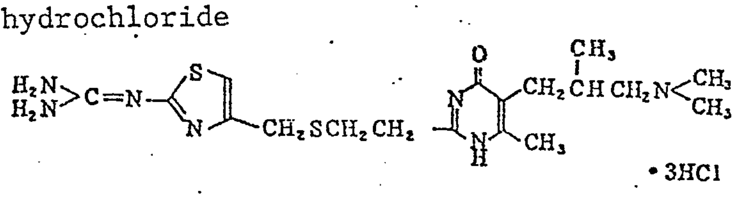

m.p. 194～198℃

Anal. ($C_{18}H_{32}N_7OS_2Cl_3$)

| | | C | H | N | S | Cl |
|---|---|---|---|---|---|---|
| Calc. | (%) | 40.56 | 6.05 | 18.40 | 12.03 | 19.96 |
| Found | (%) | 40.57 | 6.27 | 18.56 | 12.02 | 19.93 |

Example 35

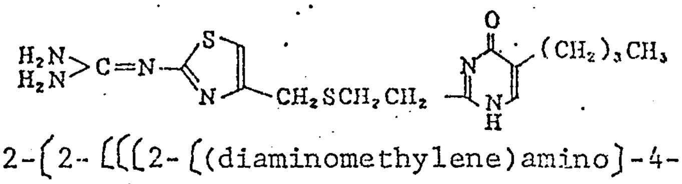

.2HCl

5-n-butyl-2-[2-[[[2-[(diaminomethylene)amino]-4-thiazolyl]-methyl] thio]ethyl]-4(1H)-pyrimidone di-hydrochloride

m.p. 163 ~ 165 ℃

Anal. ( $C_{15}H_{24}N_6OS_2Cl_2 \cdot H_2O$ )

| | | C | H | N | S | Cl |
|---|---|---|---|---|---|---|
| Calc. | (%) | 39.39 | 5.73 | 18.37 | 14.02 | 15.50 |
| Found | (%) | 39.70 | 5.89 | 18.50 | 14.34 | 15.89 |

Example 36

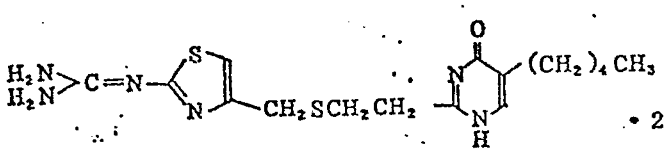

• 2HCl

2-[2-[[[2-[(diaminomethylene)amino]-4-thiazolyl]methyl] thio]-ethyl]-5-n-pentyl-4(1H)-pyrimidone di-hydrochloride

m.p. 162 ~ 164 ℃

Anal. ( $C_{16}H_{26}N_6OS_2Cl_2 \cdot H_2O$ )

| | | C | H | N | S | Cl |
|---|---|---|---|---|---|---|
| Calc. | (%) | 40.76 | 5.99 | 17.83 | 13.60 | 15.04 |
| Found | (%) | 40.57 | 5.94 | 17.70 | 13.75 | 14.74 |

Exmple 37

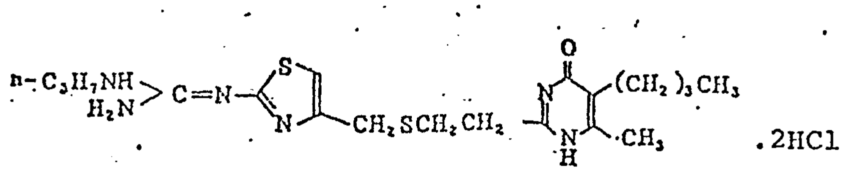

5-n-butyl-6-methyl-2-[2-[[[2- [(N-n-propyl)diaminomethylene]-amino]-4-thiazolyl]methyl]thio]ethyl]-4(1H)-pyrimidone di-hydrochloride

m.p. 163～165℃

Anal. ($C_{19}H_{32}N_6OS_2Cl_2$)

| | | C | H | N | S | Cl |
|---|---|---|---|---|---|---|
| Calc. | (%) | 46.05 | 6.51 | 16.96 | 12.94 | 14.31 |
| Found | (%) | 45.89 | 6.76 | 16.94 | 13.04 | 14.10 |

Mass. (m/z) 422($M^+$), 230

Example 38

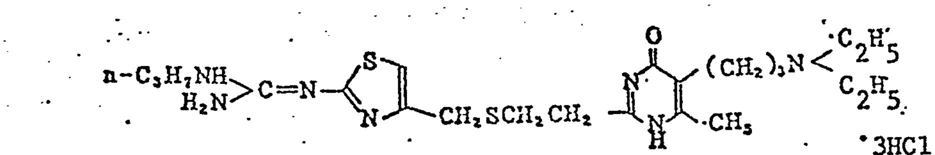

5-(3-diethylaminopropyl)-6-methyl-2-[2-[[[2-n-propyl)diaminomethylene]amino]-4-thiazolyl]methyl]thio]- ethyl]4(1H)-pyrimidone trihydrochloride

m.p. 184～187℃

Anal. ($C_{22}H_{40}N_7OS_2Cl_3$)

| | | C | H | N | S | Cl |
|---|---|---|---|---|---|---|
| Calc. | (%) | 44.86 | 6.84 | 16.64 | 10.88 | 18.06 |
| Found | (%) | 44.85 | 7.13 | 16.62 | 10.97 | 18.07 |

Mass. (m/z) 479($M^+$)

Example 39

6-Methyl-2-methyl- 3-(m-piperidinomethylphenoxy)propyl -4(1H)-pyrimidone

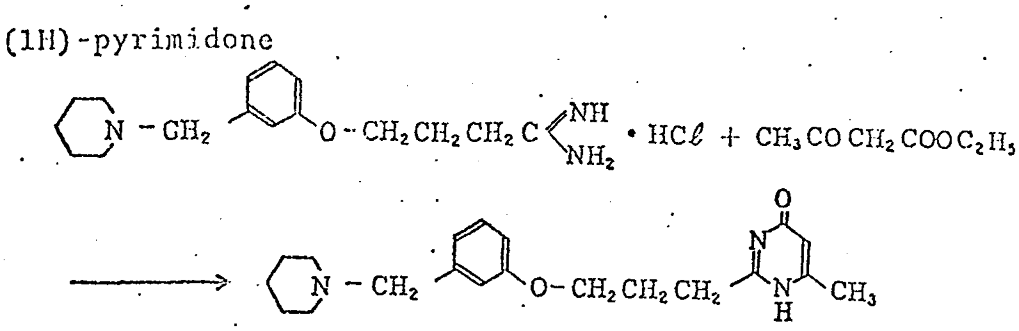

To 30 ml of dry ethanol was added metallic sodium under cooling. To the resulting solution was added gradually a solution of 3.5 g of 4-(m-piperidinomethylphenoxy)butaneamidine hydrochloride in 10 ml of dry methanol under cooling, followed by stirring for 10 minutes. Then, to the solution was added gradually a solution of 1.28 ml of ethyl acetoacetate in 10 ml of dry ethanol under cooling. After stirring for 24 hours at room temperature, the resulting salt was filtered and the solvent was distilled off under reduced pressure. The residue obtained was purified by column chromatography using chloroform - methanol as a devloping solvent, and was recrystallized from iso-propanol to provide 2.04 g of product.

m. p. 120～121 ℃ (iso — propanol)

Mass. 341 ($M^+$)

Anal. ($C_{20}H_{27}N_3O_2$)

| | C (%) | H (%) | N (%) |
|---|---|---|---|
| Calç. | 70.35 | 7.97 | 12.31 |
| Found | 70.62 | 7.92 | 12.45 |

Examples 40-47

By following the same procedure as in Example 39, the following compounds were obtained:

Example 40

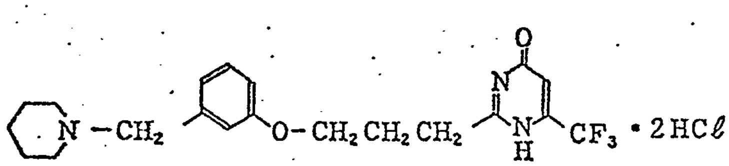

6-trifluoromethyl-2-[3-(m-piperidinomethylphenoxy)-propyl]-4(1H)-pyrimidone di-hydrochloride

m. p. 119～124℃

Mass. (m/e) 395 ($M^+$ −2HCℓ)

Anal. ($C_{20}H_{24}N_3O_2F_3$ · 2HCℓ)

| | C (%) | H (%) | N (%) | Cℓ (%) |
|---|---|---|---|---|
| Calc. | 51.29 | 5.60 | 8.97 | 15.14 |
| Found | 51.15 | 5.62 | 8.95 | 14.80 |

Example 41

5,6-dimethyl-2-{3-(m-piperidinomethylphenoxy)propyl}-4(1H)-pyrimidone di-hydrochloride

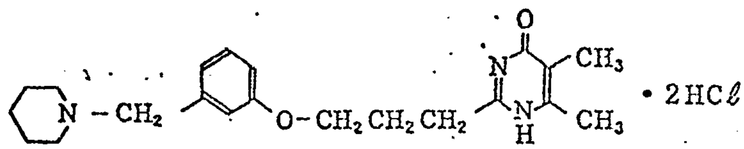

m. p. 192~195℃ (ethanol - ethyl acetate)

Mass. (m/e) 355 ($M^+$ −2HCℓ)

Anal. ($C_{21}H_{29}N_3O_2$ • 2HCℓ)

| | C (%) | H (%) | N (%) | Cℓ (%) |
|---|---|---|---|---|
| Calc. | 58.88 | 7.29 | 9.81 | 16.55 |
| Found | 58.56 | 7.27 | 9.69 | 16.38 |

Example 42

6-methyl-5-(p-phenylbenzyl)-2-[3-(m-piperidinomethylphenoxy)propyl]-4(1H)-pyrimidone di-hydrochloride

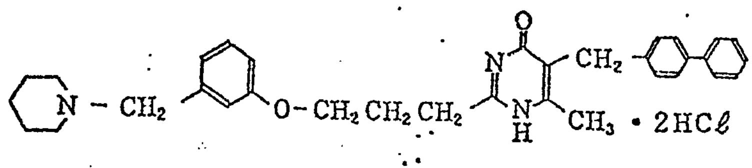

m. p. 193～196℃ (ethanol)

Mass. (m/e) 507 ($M^+$ −2HCℓ)

Anal. ($C_{33}H_{37}N_3O_2$ · 2HCℓ)

| | C (%) | H (%) | N (%) | Cℓ (%) |
|---|---|---|---|---|
| Calc. | 68.27 | 6.77 | 7.24 | 12.51 |
| Found | 67.97 | 6.76 | 7.21 | 12.38 |

Example 43

6-methyl-5-(1-naphthylmethyl)-2-[3-(m-piperidinomethylphenoxy)propyl]-4(1H)-pyrimidone di-hydrochloride

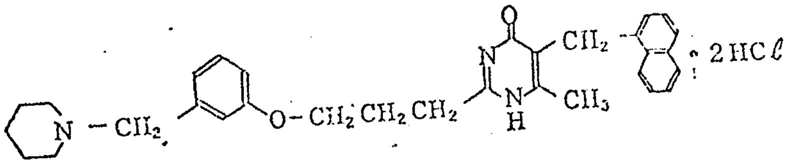

m. p. 176 ~ 178 ℃ ( ethanol)

Mass. (m/e) 481 ( $M^+ - 2HCl$ )

Anal. ( $C_{31}H_{35}N_3O_2 \cdot 2HCl$ )

| | C (%) | H (%) | N (%) | Cl (%) |
|---|---|---|---|---|
| Calc. | 67.14 | 6.72 | 7.58 | 12.79 |
| Found | 66.90 | 6.76 | 7.37 | 12.36 |

Example 44

5-[(6-chloro-1,3-benzodioxole-5-yl)methyl]-6-methyl-2-[3-(m-piperidinomethylphenoxy)propyl]-4(1H)-pyrimidone

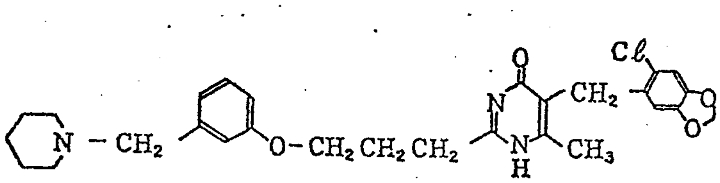

m. p. 141 ~ 142 ℃ (methanol)

Mass. (m/e) 509 ( $M^+ - 1$ )

Anal. ( $C_{28}H_{32}N_3O_4Cl$ )

| | C (%) | H (%) | N (%) | Cl (%) |
|---|---|---|---|---|
| Calc. | 65.94 | 6.32 | 8.24 | 6.95 |
| Found | 65.95 | 6.37 | 8.32 | 7.28 |

Example 45

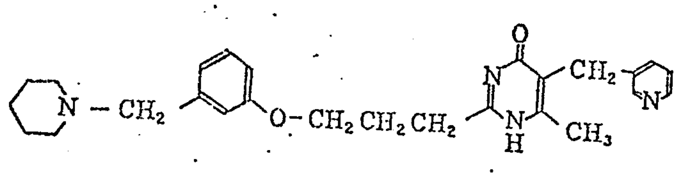

6-methyl-2-[3-(m-piperidinomethylphenoxy)propyl]-4(1H)-pyrimidone

m. p. 122～123℃ (ethanol-ether-chloroform)

Mass. (m/e) 432 ($M^+$)

Anal. ($C_{26}H_{32}N_4O_2$)

| | C (%) | H (%) | N (%) |
|---|---|---|---|
| Calc. | 72.19 | 7.46 | 12.95 |
| Found | 72.26 | 7.31 | 12.97 |

Example 46

5-(2-benzimidazolylmethyl)-6-methyl-2-[3-(m-piperidinomethylphenoxy)propyl]-4(1H)-pyrimidone

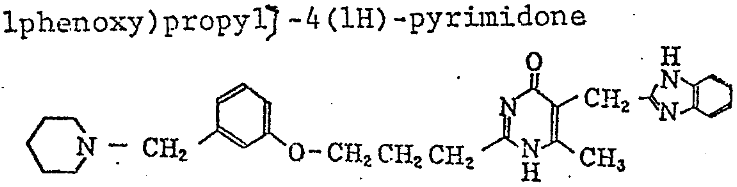

m. p. 208~209°C (methanol-ethanol)

Mass. (m/e) 471 ($M^+$)

Anal. ($C_{28}H_{33}N_5O_2$)

| | C (%) | H (%) | N (%) |
|---|---|---|---|
| Calc. | 71.31 | 7.05 | 14.85 |
| Found | 71.04 | 7.01 | 14.74 |

Example 47

5-(p-tert-butylbenzyl)-6-methyl-2-{3-(m-piperidinomethylphenoxy)propyl}-4(1H)-pyrimidone di-hydrochloride

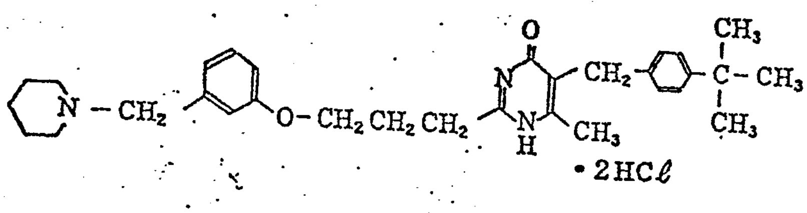

m. p. 189~193°C (ethanol- ethylacetate)

Mass. (m/e) 487 ($M^+-2HCl$)

Anal. ($C_{31}H_{41}N_3O_2 \cdot 2HCl$)

| | C (%) | H (%) | N (%) | Cl (%) |
|---|---|---|---|---|
| Calc. | 66.42 | 7.73 | 7.50 | 12.65 |
| Found | 66.26 | 7.74 | 7.50 | 12.77 |

Reference Example 39

3-[5-(dimethylaminomethyl)furfurylthiomethyl] propionamidine di hydrochloride

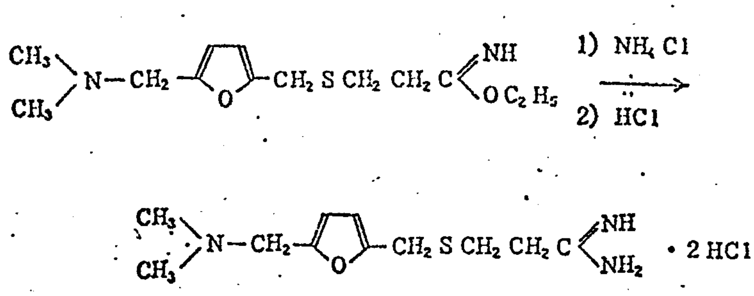


34 g of ethyl 3- 5-(dimethylaminomethyl)furfurylthiomethyl propionimidate was dissolved in 250 ml of dry methanol, and after adding 6 g of ammonium chloride and stirring the mixture at room temperature for 1 hour, an ethanol solution containing hydrochloric acid was added to the mixture to produce a dihydrochloride, and the solvent was distilled off. The residue was recrystallized from a mixture of isopropanol and ethyl acetate to provide 3- 5-(dimethylaminomethyl)furfurylthiomethyl propionamidine di-hydrochloride (31 g) having a melting point of 134 - 136°C.

Reference Example 40

Ethyl α-(3-pyridylmethyl)acetoacetate

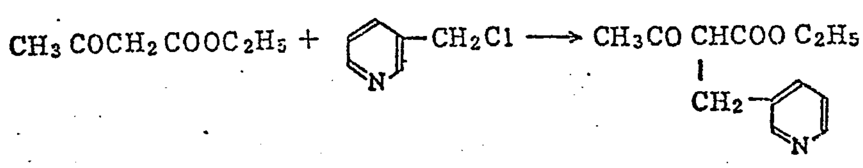

Under nitrogen, 8.4 g of metallic sodium was dissolved in 500 ml of anhydrous ethanol, and after adding 23.8 g of ethyl acetoacetate and stirring the mixture at room temperature for 1 hour, 30 g of 3-chloromethylpyridine hydrochloride was added to the mixture followed by refluxing under mild heating.

Salt formed was filtered away, and the solvent was distilled

24.07.85

away. The residue obtained was purified by column chromatography using chloroform - ethylacetate as a developing solvent to provide 12.0 g of oil product.

NMR ($CDCl_3$)

| | | |
|---|---|---|
| δ (ppm) ; | 1.18 | (3H, t) |
| | 2.20 | (3H, s) |
| | 3.13 | (2H, d) |
| | 3.78 | (1H, t) |
| | 4.13 | (2H, q) |
| | 7.04 ~ 7.60 | (2H, m) |
| | 8.40 | (2H, m) |

Mass (m/e) 221 ($M^+$)

Reference Examples 41 - 44

By following the same procedure as in Reference Example 40, the following compounds were obtained:

Reference Example 41

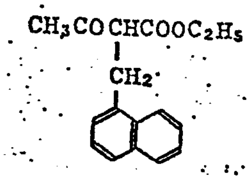

ethyl α-(1-naphthyl-methyl)-acetoacetate

NMR ($CDCl_3$)

| | | |
|---|---|---|
| δ (ppm) ; | 1.16 | (3H, t) |
| | 2.15 | (3H, s) |
| | 3.64 | (2H, d) |
| | 3.95 | (1H, t) |
| | 4.13 | (2H, q) |
| | 7.2 ~ 8.2 | (7H, m) |

Mass (m/e) 270 ($M^+$), 227, 181

Reference Example 42

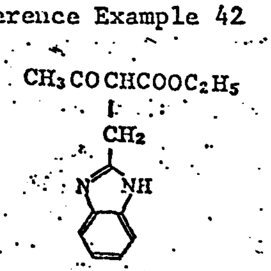

ethyl α-(2-benzimidazolyl-methyl)acetoacetate

Mass (m/e) 260 ($M^+$), 217

Reference Example 43

ethyl α-(p-tert-butylbenzyl)acetoacetate

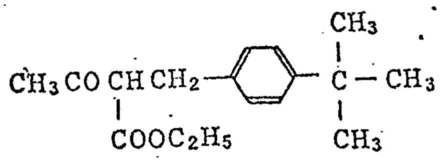

NMR($CDCl_3$)
δ(ppm); 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00~7.36 (m, 4H)

IR(neat)
ν; 1735, 1710 $cm^{-1}$

Mass (m/e)
276 ($M^+$), 261 ($M^+$−15)
233 ($M^+$− 43)

Reference Example 44

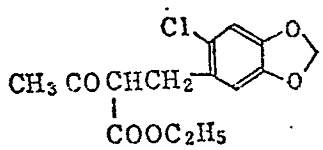

ethyl α-[(6-chloro-1,3-benzodioxole-5-yl)-methyl]ace

NMR($CDCl_3$)
δ(ppm); 1.20 (t, 3H)
2.18 (s, 3H)
2.94~3.35 (m, 2H)
3.60~3.87 (m, 1H)
4.10 (q, 2H)
5.83 (s, 2H)
6.54~6.81 (m, 2H)

IR(neat)
ν; 1735, 1710 $cm^{-1}$

Mass (m/e)
298 ($M^+$), 255 ($M^+$−43)

Example 48

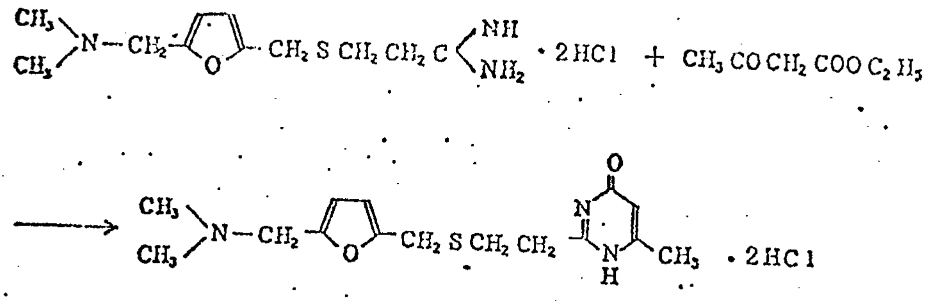

To 30 ml of ethanol containing 0.44 g of metallic sodium was added 3.0 g of 3-[5-(dimethylaminomethyl)furfurylthio]-propionamidine di-hydrochloride followed by stirring for 10 minutes, and to the solution was added 1.24 g of ethyl acetoacetate followed by stirring for 20 hours at room temperature. The solvent was distilled off under reduced pressure. The residue was purified by column chromatography using chloroform - methanol mixture as a developing solvent. After converting the product to the di-hydrochloride with ethanol containing hydrochloric acid, the product was recrystallized from ethanol to provide 2- 2- 5-(dimethylaminomethyl)furfurylthio ethyl -6-methyl-(1H)-pyrimidone di-hydrochloride (1.7 g).

m. p. 163 ~ 165℃ (ethanol)

Anal ($C_{15}H_{23}N_3O_2S \cdot Cl_2$)

| | C (%) | H (%) | N (%) |
|---|---|---|---|
| Calc. | 47.37 | 6.10 | 11.05 |
| Found | 47.14 | 5.91 | 10.98 |

Examples 49 - 54

By following the same procedure as in Example 48, the following compounds were obtained:

Example 49

2-[2-[[5-(dimethylaminomethyl)furfurylthio] ethyl]-5,6-dimethyl-4(1H)-pyrimidone di-hydrochloride

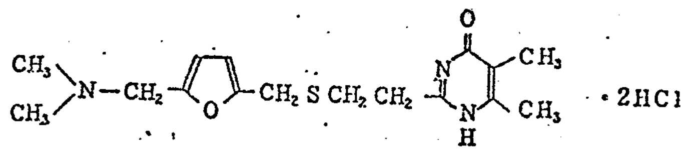

m.p. 172 ~ 177°C (ethanol - ethyl actoacetate)

Anal. ($C_{16}H_{25}N_3O_2SCl_2$)

| | C (%) | H (%) | N (%) |
|---|---|---|---|
| Calc. | 48.73 | 6.39 | 10.66 |
| Found | 48.44 | 6.27 | 10.49 |

Example 50

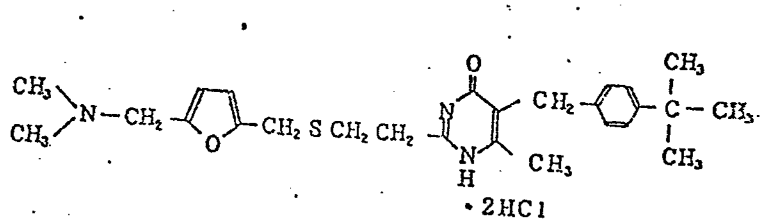

5-(p-tert-butylbenzyl)-2-[2-[5-(dimethylaminomethyl)furfurylthio] ethyl]-6-methyl-4(1H)-pyrimidone di-hydrochloride

m.p. 171 ~ 175°C (ethanol)

Anal. ($C_{26}H_{37}N_3O_2SCl_2$)

| | C (%) | H (%) | N (%) |
|---|---|---|---|
| Calc. | 59.31 | 7.08 | 7.98 |
| Found | 59.12 | 7.14 | 7.94 |

Example 51

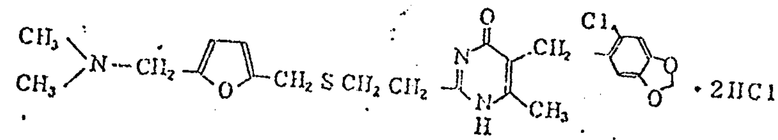

5-[(6-chloro-1,3-benzodioxole-5-yl)methyl]-2-[2-[5-(dimethylaminomethyl)furfurylthio]ethyl]-6-methyl-4(1H)-pyrimidone di-hydrochloride

m. p. 158 ~ 162 ℃ (ethanol)

Anal. ($C_{23}H_{28}N_3O_4SCl_3$)

| | C (%) | H (%) | N (%) |
|---|---|---|---|
| Calc. | 50.33 | 5.14 | 7.66 |
| Found | 50.27 | 5.07 | 7.55 |

Example 52

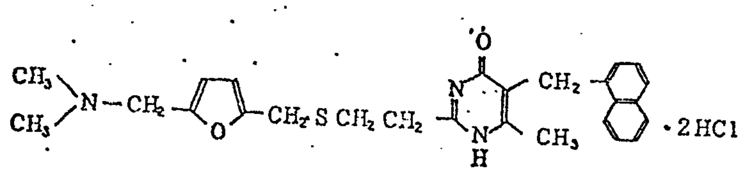

2-[2-[5-(dimethylaminomethyl)furfurylthio]ethyl]-6-methyl-5-(1-naphthylmethyl)-4(1H)-pyrimidone di-hydrochloride

m. p. 178 ~ 181 ℃ (ethanol)

Anal. ($C_{26}H_{31}N_3O_2SCl_2 \cdot 1/2 H_2O$)

| | C (%) | H (%) | N (%) |
|---|---|---|---|
| Calc. | 58.97 | 6.09 | 7.94 |
| Found | 59.15 | 5.97 | 7.94 |

Example 53

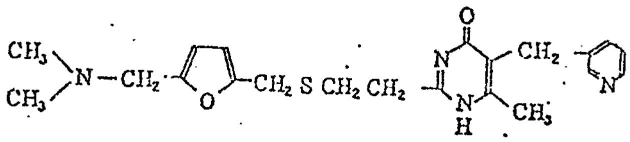

•3HCl

2-[2-[5-(dimethylaminomethyl)furfurylthio]ethyl]-6-methyl-5-(3-pyridylmethyl)-4(1H)-pyrimidone trihydrochloride

m.p. 164 ~ 166 ℃ (ethanol)

Anal. ( $C_{21}H_{29}N_4O_2SCl_3$ )

| | C (%) | H (%) | N (%) |
|---|---|---|---|
| Calc. | 49.66 | 5.75 | 11.03 |
| Found | 49.26 | 5.83 | 10.78 |

Example 54

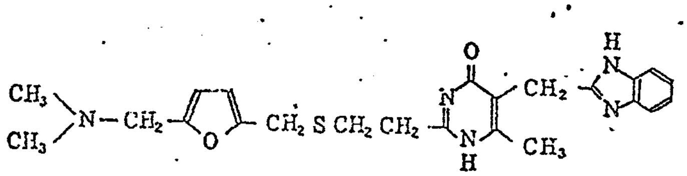

•3HCl

5-(2-benzimidazolylmethyl)-2-[2-[5-(dimethylaminomethyl)-furfurylthio]ethyl]-6-methyl-4(1H)-pyrimidone tri-hydrochloride

m.p. 185 ~ 189 ℃ (ethanol)

Anal. ( $C_{23}H_{30}N_5O_2SCl_3$ )

| | C (%) | H (%) | N (%) |
|---|---|---|---|
| Calc. | 50.51 | 5.53 | 12.80 |
| Found | 50.22 | 5.56 | 12.64 |

Example 55

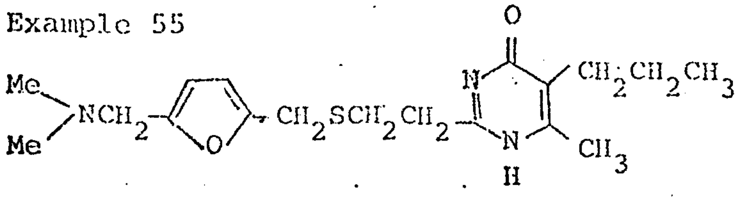

.2HCl

mp. 150 - 155°C

Mass. (m/z) 350 ($M^+$+1), 304 ($M^+$-45)

Example 56

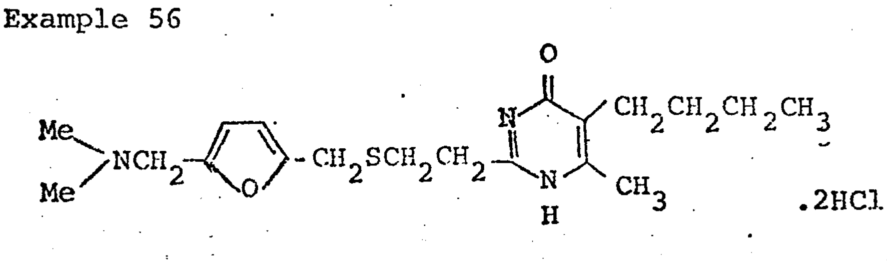

.2HCl

mp. 158 -162°C

Mass. (m/z) 364 ($M^+$+1), 318 ($M^+$-45)

Anal. ($C_{19}H_{31}N_3O_2SCl_2$)

| | C | H | N | S | Cl |
|---|---|---|---|---|---|
| Cal. (%) | 52.29 | 7.16 | 9.63 | 7.35 | 16.25 |
| Found (%) | 52.03 | 7.45 | 9.64 | 7.29 | 16.22 |

Example 57

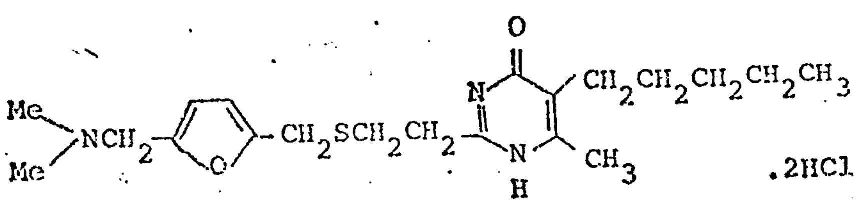

.2HCl

mp. 153 - 156°C

Mass. (m/z) 378 ($M^{+}$+1), 332 ($M^{+}$-45)

Anal. ($C_{20}H_{33}N_{3}O_{2}SCl_{2}$)

| | C | H | N | S | Cl |
|---|---|---|---|---|---|
| Calc. (%) | 53.33 | 7.38 | 9.33 | 7.12 | 15.74 |
| Found (%) | 53.28 | 7.56 | 9.38 | 7.26 | 15.57 |

Example 58

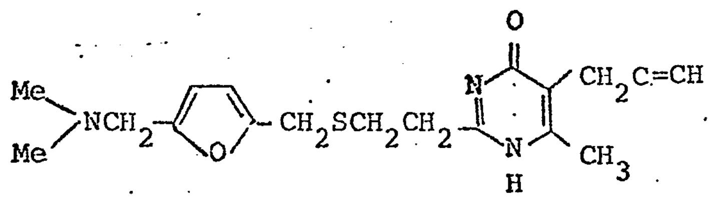

mp. 98 -98.5°C

Mass. (m/z) 346 ($M^{+}$+1), 300 ($M^{+}$-45)

Anal. ($C_{18}H_{23}N_{3}O_{2}S$)

| | C | H | N | S |
|---|---|---|---|---|
| Calc. (%) | 62.58 | 6.71 | 12.16 | 9.28 |
| Found (%) | 62.67 | 6.42 | 12.20 | 9.25 |

By following the same procedure as in Example 39, the following compounds were obtained

Example 59

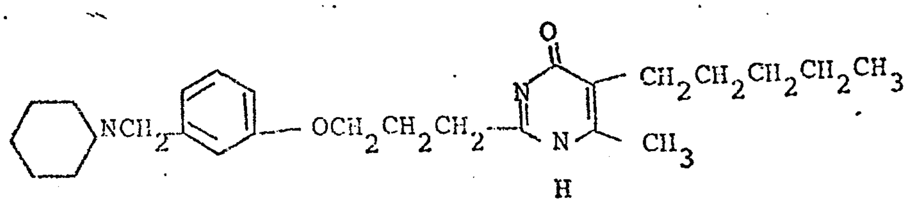

mp. 80 - 82°C

Mass(m/z) 412($M^{+}$+1)

Anal.($C_{25}H_{37}N_3O_2$)

| | C | H | N |
|---|---|---|---|
| Calc. (%) | 72.96 | 9.06 | 10.21 |
| Found (%) | 72.75 | 9.08 | 10.16 |

Example 60

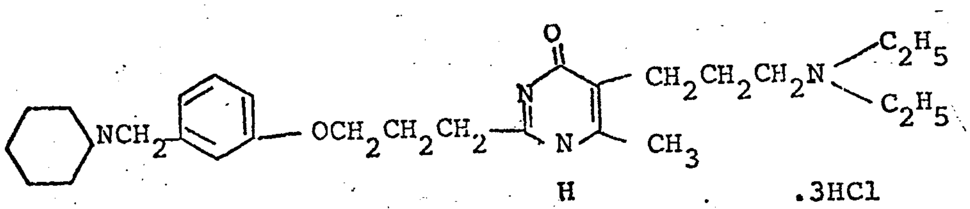

.3HCl

mp. 128 - 130°C

Mass.(m/z) 454($M^{+}$), 425($M^{+}$-29)

By following the same procedure as in Example 1, the following compounds were obtained.

Example 61

6-Methyl-2-[2-([[2-([(n-propyl)diaminomethylene]amino]-4-thiazolyl]-methyl] thio) ethyl]-4(1H)-pyrimidone·di-hydrochloride

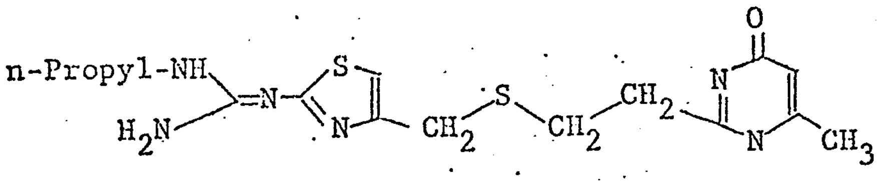

;2HCl

Mass(m/z) 366($M^+$)

mp. 165.5 - 168°C (ethanol-ethyl acetate)

Anal. ($C_{15}H_{22}N_6OS_2$ 2HCl 1/2$H_2$)

| | C | H | N | S | Cl |
|---|---|---|---|---|---|
| Calc.(%) | 40.18 | 5.62 | 18.74 | 14.30 | 15.81 |
| Found(%) | 40.45 | 5.34 | 19.00 | 14.71 | 15.94 |

Example 62

5-Ethyl-6-methyl-2-[2-([[2-[(n-propyl)diaminomethylene]-amino]-4-thiazolyl]methyl]thio]ethyl]-4(1H)-pyrimidone dihydrochloride

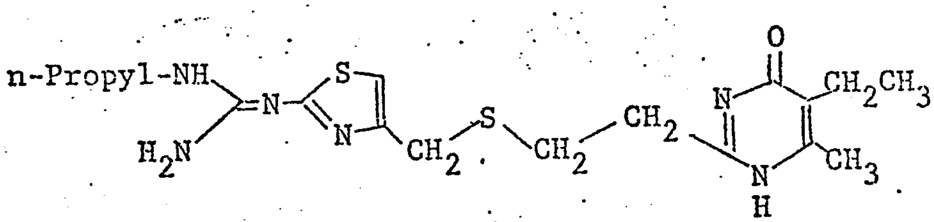

.2HCl

mp. 155 - 157°C (ethanol-ethyl acetate)

Mass.(m/z) 394($M^+$)

Anal. ($C_{17}H_{26}N_6OS_2 \cdot 2HCl$)

| | C | H | N | S |
|---|---|---|---|---|
| Calc.(%) | 43.68 | 6.04 | 17.98 | 13.72 |
| Found.(%) | 43.50 | 6.05 | 18.01 | 13.68 |

Example 63

5-n-Hexyl-6-methyl-2-[2-[[[2-[[(n-propyl)diaminomethylene]-amino]-4-thiazolyl]methyl]thio]ethyl]-4(1H)pyrimidone·di-hydrochloride

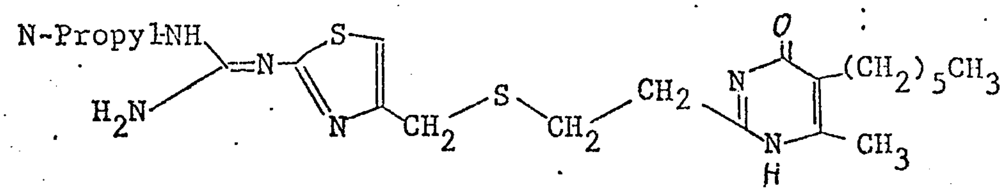

·2HCl

mp. 148 - 150°C (ethanol-ethyl acetate)

Mass.(m/z) 450($M^+$)

Anal. ($C_{21}H_{34}N_6OS_2 \cdot 2HCl$)

| | C | H | N | S | Cl |
|---|---|---|---|---|---|
| Calc.(%) | 48.17 | 6.93 | 16.05 | 12.25 | 13.54 |
| Found (%) | 47.98 | 6.71 | 16.09 | 12.12 | 13.64 |

Example 64

5-Benzyl-6-methyl-2-[2-[[[2-[[(n-propyl)diaminomethylene]-amino]-4-thiazolyl]methyl]thio]ethyl]-4(1H)-pyrimidone di-hydrochloride

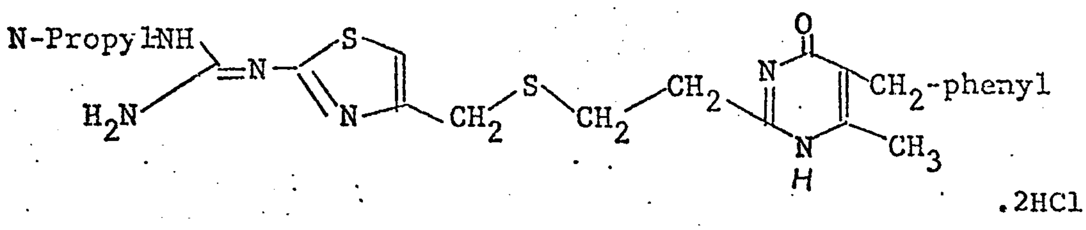

.2HCl

mp. 162 - 165°C (ethanol - ethyl acetate)

Mass. (m/z) 456($M^+$)

Anal. ($C_{22}H_{28}N_6OS_2 \cdot 2HCl$)

| | C | H | N | S | Cl |
|---|---|---|---|---|---|
| Calc.(%) | 49.90 | 5.71 | 15.87 | 12.11 | 13.39 |
| Found (%) | 49.71 | 5.51 | 15.69 | 11.87 | 13.52 |

C L A I M S :

1. A compound of the formula I, or a pharmacologically acceptable salt thereof:

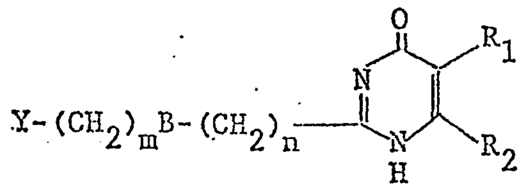

(I)

wherein $R_1$ represents a hydrogen atom, a $C_1$ to $C_{10}$ alkyl group, a $C_1$ to $C_5$ alkenyl group, a $C_1$ to $C_5$ alkinyl group, a phenyl group, a cycloalkylmethyl group, or an unsubstituted or substituted aralkyl group; $R_2$ represents a hydrogen atom, a $C_1$ to $C_5$ alkyl group or a trifluoromethyl group; Y represents a heterocyclic group of the formula:

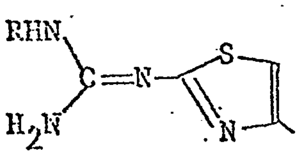

,

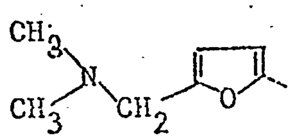

or

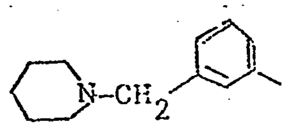

wherein R represents a hydrogen atom or a $C_1$ to $C_5$ alkyl group; B represents an oxygen atom or a sulfur atom; m represents zero or an integer of 1 to 3; and n represents an integer of 1 to 3.

2. A compound according to claim 1 wherein $R_1$ is an aralkyl group in which the aryl portion is optionally substituted by at least one substituent selected from $C_1$ to $C_5$ alkyl groups, $C_1$ to $C_5$ alkoxy groups, halogens and $C_1$ to $C_5$ alkylenedioxy and phenyl groups.

3. A compound according to claim 1 wherein B is oxygen and m is zero.

4. A compound according to claim 1 wherein B is sulfur and m and n are the same or different integers of 1 to 3.

5. A compound according to claim 1 wherein Y is

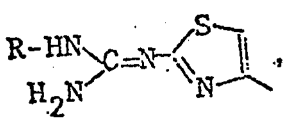

, B is sulfur, and m and n are the same or different integers of 1 to 3.

6. A compound according to claim 6 wherein R is a $C_1$ to $C_3$ alkyl group.

7. A compound according to claim 1 wherein Y is

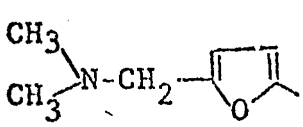

, and m and n are the same or

different integers of 1 to 3.

8. A compound according to claim 7 wherein m is 1 and n is 2 and $R_1$ is an aralkyl group selected from naphthylmethyl, pyridylmethyl, benzimidazolylmethyl and phenylmethyl groups which are optionally substituted on the aryl ring by at least one substituent selected from halogens and tert-butyl, methylenedioxy and phenyl groups.

9. A compound according to claim 1 wherein Y is

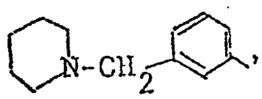, B is oxygen, and m is zero.

10. A compound according to claim 9 wherein n is 3 and $R_1$ is an aralkyl group selected from naphthylmethyl, pyridylmethyl, benzimidazolylmethyl and phenylmethyl groups which are optionally substituted on the aryl ring by at least one substituent selected from halogens and tert-butyl, methylenedioxy and phenyl groups.

11. A compound according to claim 1 wherein $R_1$ is a $C_4$ to $C_9$ alkyl group.

12. One or more of the compounds of claim 1 :

5-(p-tert-butylbenzyl)-2-[2-([[2-[(diaminomethylene)amino]-4-thiazolyl]methyl]thio]ethyl]-6-methyl-4(1H)-pyrimidone;

2-[2-([[2-[(diaminomethylene)amino]-4-thiazolyl]methyl]thio]-ethyl]-5-n-butyl-6-methyl-4(1H)-pyrimidone;

2-[2-([[2-[(diaminomethylene)amino]-4-thiazolyl]methyl]thio]-ethyl]-6-methyl-5-n-pentyl-4(1H)-pyrimidone;

2-[2-[[[2-[(diaminomethylene)amino]-4-thiazolyl]methyl]thio]-ethyl]-6-methyl-5-(1-naphthylmethyl)-4(1H)-pyrimidone;

5-[(1,3-benzodioxole-5-yl)methyl]-2-[2-[5-(dimethylaminomethyl)furfurylthio]ethyl]-6-methyl-4(1H)-pyrimidone;

5-(p-tert-butylbenzyl)-6-methyl-2-[3-(m-piperidinomethyl-phenoxy)propyl]-4(1H)-pyrimidone; and salts thereof.

13. A pharmaceutical composition containing a compound according to any preceding claim and excipient.

14. A process for preparing a compound according to claim 1 which comprises reacting a compound of the

formula :

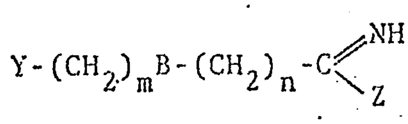

with a compound of the formula :

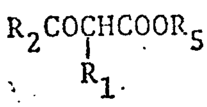 or 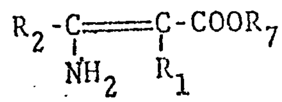

wherein Y, B, m, n, $R_1$ and $R_2$ are as defined in claim 1, $R_5$ is a $C_1$ to $C_5$ alkyl group, $R_7$ is a $C_1$ to $C_5$ alkyl group, and Z is $NH_2$ or $OR_6$ wherein $R_6$ is a $C_1$ to $C_5$ alkyl group.

15. A process according to claim 14 which comprises reacting a compound of the formula:

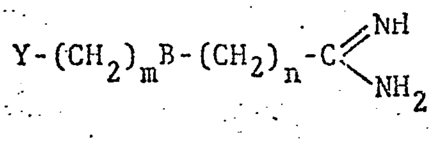

wherein Y, B, m and n are as defined in claim 14 with a compound of the formula

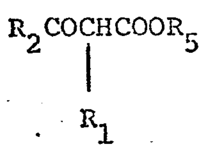

wherein $R_1$, $R_2$ and $R_5$ are as defined in claim 14.

16. A process according to claim 14 which comprises reacting a compound of the formula :

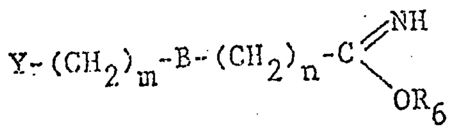

wherein Y, B, $R_6$, m and n are as defined in claim 14 with a compound of the formula :

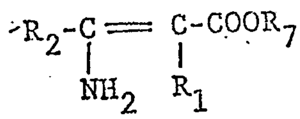

wherein $R_1$, $R_2$, and $R_7$ are as defined in claim 14.